(19) Europäisches Patentamt
European Patent Office
Office européen des brevets

(11) **EP 4 303 581 B1**

(12) **EUROPÄISCHE PATENTSCHRIFT**

(45) Veröffentlichungstag und Bekanntmachung des Hinweises auf die Patenterteilung:
**17.07.2024   Patentblatt 2024/29**

(21) Anmeldenummer: **22207091.4**

(22) Anmeldetag: **14.11.2022**

(51) Internationale Patentklassifikation (IPC):
***G01N 33/18*** (2006.01)   ***G06F 17/00*** (2019.01)

(52) Gemeinsame Patentklassifikation (CPC):
**G01N 33/18;** G06F 17/18; Y02A 20/152

(54) **SYSTEM UND VERFAHREN ZUR INTELLIGENTEN FRÜHWARNUNG VOR WASSERBLÜTEN BASIEREND AUF DER VORHERSAGE DES ERNÄHRUNGSZUSTANDS VON GEWÄSSERN**

SYSTEM AND METHOD FOR INTELLIGENT EARLY WARNING OF WATER FLOWERING BASED ON PREDICTION OF NUTRITIONAL STATE OF WATER BODIES

SYSTÈME ET PROCÉDÉ D'ALERTE PRÉCOCE INTELLIGENTE DE FLEURS AQUATIQUES SUR LA BASE D'UNE PRÉDICTION DE L'ÉTAT NUTRITIONNEL DES EAUX

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **05.07.2022   CN 202210780993**

(43) Veröffentlichungstag der Anmeldung:
**10.01.2024   Patentblatt 2024/02**

(73) Patentinhaber: **China Three Gorges Co., Ltd. Wuhan Hubei 430010 (CN)**

(72) Erfinder:
• **DAI, Huichao**
  **Wuhan, 430010 (CN)**
• **MAO, Jinqiao**
  **Wuhan, 430010 (CN)**
• **CHEN, Yanhong**
  **Wuhan, 430010 (CN)**
• **ZHU, Shijie**
  **Wuhan, 430010 (CN)**
• **JIANG, Dingguo**
  **Wuhan, 430010 (CN)**
• **DAI, Jie**
  **Wuhan, 430010 (CN)**
• **WANG, Kang**
  **Wuhan, 430010 (CN)**

(74) Vertreter: **Herrmann, Uwe
Lorenz Seidler Gossel
Rechtsanwälte Patentanwälte
Partnerschaft mbB
Widenmayerstraße 23
80538 München (DE)**

(56) Entgegenhaltungen:
CN-A- 106 990 216   CN-A- 110 887 790
CN-A- 111 242 380   CN-U- 203 708 656

• GUAN WEIBING ET AL: "Monitoring, modeling and projection of harmful algal blooms in China", HARMFUL ALGAE, ELSEVIER, AMSTERDAM, NL, vol. 111, 22 December 2021 (2021-12-22), XP086919394, ISSN: 1568-9883, [retrieved on 20211222], DOI: 10.1016/J.HAL.2021.102164

EP 4 303 581 B1

**Beschreibung**

Gebiet der Erfindung

Die vorliegende Anmeldung bezieht sich auf das technische Gebiet der Umweltüberwachung, insbesondere auf ein System und ein Verfahren zur intelligenten Frühwarnung vor Wasserblüten basierend auf der Vorhersage des Ernährungszustands von Gewässern.

Stand der Technik

Unter dem Einfluss von Wasserschutzprojekten ändert sich die Hydrodynamik von Wasserkörpern in empfindlichen Gewässern wie Nebenflüssen von Stauseen, Lebensräumen für aquatische Organismen, Flüssen, Ufern, Seen und Feuchtgebieten und der vertikale Austausch von Wasserkörpern wird unterdrückt. Wenn eine große Menge an Nährstoffen in den Wasserkörper fließt, ist es leicht, den Wasserkörper von einem oligotrophen Zustand in einen eutrophen Zustand zu verwandeln. Unter bestimmten Umweltbedingungen kann eine übermäßige Eutrophierung von Wasserkörpern zu Wasserblütenausbrüchen führen. Wenn die Wasserblüte ausbricht, führt dies zu einer Verschlechterung der Wasserqualität und einem ökologischen Ungleichgewicht des Wasserkörpers, was eine ernsthafte Bedrohung für die Trinkwassersicherheit und die aquatischen Ökosysteme darstellt. Daher ist es notwendig, den Zustand von Wasserkörpern in empfindlichen Gewässern zu überwachen, um das Auftreten von Wasserblüten zu vermeiden.

In einigen verwandten Wasserkörperüberwachungstechnologien werden die meisten von ihnen durch Fernerkundungssatelliten, Drohnenfotografie oder manuelle Probenahmetests überwacht, so dass im Allgemeinen nur der Zustand von Oberflächenwasserkörpern identifiziert werden kann.

Eine Übersicht verschiedener Möglichkeiten zur Erkennung von Bedingungen, die zu einer Wasserblüte führen können, insbesondere Eutrophierung, ist beispielsweise GUAN WEIBING, "Monitoring, modeling and projection of harmful algal blooms in China" (2021) zu entnehmen.

Das Dokument CN111242380 offenbart die Nutzung künstlicher Intelligenz, insbesondere die Verwendung maschineller Lernalgorithmen, zur Vorhersage der Eutrophierung von Gewässern an Hand von Wasserqualitätsdaten, darunter insbesondere Daten zu Temperatur, Trübung, Leitfähigkeit und Chlorophyllgehalt des Wassers.

Die Fernerkundungsüberwachung hat Zeitintervalle, in denen Überwachungsdaten fehlen. Die mangelnde Integrität der Überwachungsdaten und der komplexe Mechanismus des Auftretens von Wasserblüten erschweren eine umfassende Überwachung des Ernährungszustands von Wasserkörpern. Daher ist es unmöglich, den Ernährungszustand des Wasserkörpers präzis zu identifizieren, was die Praktikabilität der Lösung schlecht macht.

Aufgabe der Erfindung

Vor diesem Hintergrund wird gemäß den Ausführungsbeispielen der vorliegenden Anmeldung ein System und ein Verfahren zur intelligenten Frühwarnung vor Wasserblüten basierend auf der Vorhersage des Ernährungszustands von Gewässern bereitgestellt, so dass der Wasserkörper in den Zielgewässern in Echtzeit überwacht und der Ernährungszustand des Wasserkörpers in den Zielgewässern effizient und präzis identifiziert und bestimmt werden kann.

Gemäß dem ersten Aspekt wird gemäß den Ausführungsbeispielen der vorliegenden Anmeldung ein System zur intelligenten Frühwarnung vor Wasserblüten basierend auf der Vorhersage des Ernährungszustands von Gewässern bereitgestellt, umfassend die folgenden Komponenten:

ein Multiskaleninformation-Erfassungsmodul zum Sammeln der Multiskaleninformation des Wasserkörpers in dem Zielgewässer, wobei die Multiskaleninformation des Wasserkörpers physikalische und chemische Information des Wasserkörpers, Oberflächeninformation des Wasserkörpers und Referenzhydrologieinformation umfasst,

ein Ernährungszustand-Erkennungsmodul zum Invertieren und Bestimmen der Oberflächennährstoffkonzentration basierend auf der Oberflächeninformation des Wasserkörpers, zum Vorhersagen der Vertikalnährstoffkonzentration des Wasserkörpers in dem Zielgewässer basierend auf der Oberflächennährstoffkonzentration durch eine Beziehungsfunktion zwischen der Oberflächenkonzentration und der Vertikalkonzentration und zum Bestimmen des Ernährungszustands des Wasserkörpers in dem Zielgewässer basierend auf der Oberflächennährstoffkonzentration und der Vertikalnährstoffkonzentration, wobei die Beziehungsfunktion zwischen der Oberflächenkonzentration und der Vertikalkonzentration gemäß der Multiskaleninformation des Wasserkörpers angepasst und bestimmt wird,

ein Wasserblüten-Frühwarnungsmodul zum Vorhersagen der Wahrscheinlichkeit des Auftretens von Wasserblüten gemäß dem Ernährungszustand des Wasserkörpers und zum Erzeugen der entsprechenden Warnrückkopplungsbenachrichtigungsinformation gemäß der Wahrscheinlichkeit des Auftretens von Wasserblüten, und

Ein Digitalvisualisierungsmodul zum digitalen Anzeigen der Visualisierung des Ernährungszustands des Wasserkörpers, der Wahrscheinlichkeit des Auftretens von Wasserblüten und der Warnrückkopplungsbenachrichtigungsinformation.

Vorzugsweise ist vorgesehen, dass das Multiskaleninformation-Erfassungsmodul eine Wasserkörpersüberwachungseinheit, eine Telemetriedateneinheit und eine Referenzinformationssammeleinheit umfasst, Dass die Wasserkörpersüberwachungseinheit zum Erhalten von Hydrodynamikdaten, Wassertemperaturdaten, Wasserqualitätsdaten und Gewässerwinddaten des Wasserkörpers in dem Zielgewässer ausgebildet ist, dass die physikalische und chemische Information des Wasserkörpers Hydrodynamikdaten, Wassertemperaturdaten, Wasserqualitätsdaten und Gewässerwinddaten des Wasserkörpers umfasst,

dass die Telemetriedateneinheit zum Erhalten von Bilddaten in geringer Höhe und Fernerkundungsdaten in großer Höhe des Zielgewässers ausgebildet ist, dass die Oberflächeninformation des Wasserkörpers die Bilddaten in geringer Höhe und die Fernerkundungsdaten in großer Höhe umfasst,
Dass die Referenzinformationssammeleinheit zum Sammeln der entsprechenden Hydrologie- und Hydrodynamikdaten, Gewässerumweltdaten, Topographie- und Geomorphologiedaten sowie Meteorologiedaten des Zielgewässers ausgebildet ist, dass die Referenzhydrologieinformation die Hydrologie- und Hydrodynamikdaten, die Gewässerumweltdaten, die Topographie- und Geomorphologiedaten sowie die Meteorologiedaten umfasst.

Vorzugsweise ist vorgesehen, dass die Wasserkörpersüberwachungseinheit eine Unterwasser-Multifunktionsdetektionsvorrichtung und eine Überwachungsstation für physikalische und chemische Parameter umfasst, dass die Unterwasser-Multifunktionsdetektionsvorrichtung zum Erhalten von den Hydrodynamikdaten, den Wassertemperaturdaten und den Wasserqualitätsdaten ausgebildet ist, dass die Überwachungsstation für physikalische und chemische Parameter zum ergänzenden Überwachen der Wasserqualitätsdaten ausgebildet ist,

dass die Unterwasser-Multifunktionsdetektionsvorrichtung eine hydrodynamische Induktionssonde, einen Temperatursensor und einen Wasserfarbbildgeber umfasst,
Wobei die hydrodynamische Induktionssonde am Unterteil der Unterwasser-Multifunktionsdetektionsvorrichtung angeordnet ist, um hydrodynamische Faktoren mit unterschiedlichen Detektionstiefen zu detektieren und aufzuzeichnen,
Dass der Temperatursensor zum Sammeln der Wassertemperaturen von Wasserkörpern mit unterschiedlichen Detektionstiefen ausgebildet ist,
Dass der Wasserfarbbildgeber zum Fotografieren an verschiedenen Detektionstiefen im Wasserkörper und zum Detektieren und Bestimmen von Sauerstoffauflösungsdaten, Chlorophylldaten, Algendichtedaten, Stickstoffdaten, Phosphordaten und Transparenzdaten im Wasserkörper ausgebildet ist, dass die Wasserqualitätsdaten Sauerstoffauflösungsdaten, Chlorophylldaten, Algendichtedaten, Stickstoffdaten, Phosphordaten und Transparenzdaten im Wasserkörper umfassen,
wobei der Wasserfarbbildgeber zwei symmetrisch verteilte Wasserfarbbildgebungsplatten umfasst,
dass der Abstand zwischen den zwei Wasserfarbbildgebungsplatten gemäß der Detektionstiefe eingestellt wird,

$$w = \xi \sigma_i \frac{h}{e^{\lambda} + \eta}$$

Dabei $w$ den Abstand zwischen den zwei Wasserfarbbildgebungsplatten darstellt, $\xi$ den Turbulenzkorrekturkoeffizienten darstellt, die Turbulenzintensität des Wasserflusses am i Detektionspunkt darstellt,
$h$ die Detektionstiefe entsprechend dem i Detektionspunkt darstellt, $\lambda$ den spektralen Auflösungsfaktor darstellt und $\eta$ den Korrekturparameter darstellt. Vorzugsweise ist vorgesehen, dass das Ernährungszustand-Erkennungsmodul eine Oberflächenzustandsidentifikationseinheit, eine Funktionsanpassungseinheit, eine Vertikalzustandsvorhersageeinheit und eine Zustandsbestimmungseinheit umfasst,
dass die Oberflächenzustandsidentifikationseinheit zum Invertieren und Identifizieren des Ernährungszustands des Oberflächenwasserkörpers in dem Zielgewässer gemäß der Oberflächeninformation des Wasserkörpers ausgebildet ist, um die Oberflächennährstoffkonzentration zu bestimmen,
dass die Funktionsanpassungseinheit zum Konstruieren des Trainingsdatensatzes unter Verwendung der Multiskaleninformation des Wasserkörpers ausgebildet ist, dass das Training durch einen maschinellen Lernalgorithmus durchgeführt wird, um die Beziehungsfunktion zwischen der Oberflächenkonzentration und der Vertikalkonzentration anzupassen und zu bestimmen,
dass die Beziehungsfunktion zwischen der Oberflächenkonzentration und der Vertikalkonzentration ausgebildet ist, um eine Beziehung zwischen der Nährstoffkonzentration in dem Oberflächenwasserkörper des Zielgewässers und der Nährstoffkonzentration in dem Vertikalwasserkörper des Zielgewässers zu beschreiben, die Beziehungsfunktion zwischen der Oberflächenkonzentration und der Vertikalkonzentration:

$$F = \left( \mathrm{T}, c_j^s, c_j^n, u, h, D_s \right)$$

dabei $F$ die Beziehungsfunktion zwischen der Oberflächenkonzentration und der Vertikalkonzentration darstellt, T die Wasserkörperstemperatur darstellt, $c_j^s$ die Konzentration des $j$ Nährstoffs in dem Oberflächenwasserkörper darstellt, $c_j^n$ die Konzentration des $j$ Nährstoffs in dem Vertikalwasserkörper darstellt, $u$: die Strömungsgeschwindigkeit des Wasserflusses darstellt, $h$ die Wassertiefe darstellt und $D_s$ den Komponentenausdehnungskoeffizienten darstellt,

dass die Vertikalzustandsvorhersageeinheit zum Durchführen einer Vorhersageberechnung unter Verwendung der Oberflächennährstoffkonzentration als Eingabe gemäß der Beziehungsfunktion zwischen der Oberflächenkonzentration und der Vertikalkonzentration ausgebildet ist, um die Vertikalnährstoffkonzentration zu bestimmen,

Dass die Zustandsbestimmungseinheit zum Analysieren und Bestimmen des Ernährungszustands des Wasserkörpers in dem Zielgewässer basierend auf der Oberflächennährstoffkonzentration und der Vertikalnährstoffkonzentration ausgebildet ist.

Vorzugsweise ist vorgesehen, dass die Oberflächenzustandsidentifikationseinheit ein Datenverbesserungsmodul umfasst,

dass das Datenverbesserungsmodul zum Vorhersagen und Berechnen der mit dem fehlenden Periode der Oberflächeninformation des Wasserkörpers entsprechenden Oberflächennährstoffkonzentration bei der Bestimmung der Oberflächennährstoffkonzentration ausgebildet ist,

$$c_{t+\Delta t} = \psi \left( \frac{u}{\sqrt{S} D_s} \Delta t \right) c_t$$

dabei $c_{t+\Delta t}$ die Oberflächennährstoffkonzentration zum Zeitpunkt $t + \Delta t|$ darstellt, $c_t$ die Oberflächennährstoffkonzentration zum Zeitpunkt $t$ darstellt, $\Delta t$ die fehlende Zeit darstellt, $\psi$ den Korrekturkoeffizienten der Nährstoffkonzentration darstellt und $S$ die Oberflächenfläche des Zielgewässers darstellt,

Vorzugsweise ist vorgesehen, dass die Funktionsanpassungseinheit ein Kandidatenfunktionsmodul und ein Funktionsfiltermodul umfasst,

dass das Kandidatenfunktionsmodul zum Durchführen eines Lerntrainings gemäß dem Trainingsdatensatz ausgebildet ist, um mehrere Kandidatenbeziehungsfunktionen zu bestimmen,

dass das Funktionsfiltermodul zum Berechnen und Bestimmen eines Bestimmungskoeffizienten mehrerer Kandidatenbeziehungsfunktionen ausgebildet ist, um eine optimale Funktion als die Beziehungsfunktion zwischen der Oberflächenkonzentration und der Vertikalkonzentration gemäß dem Bestimmungskoeffizienten auszuwählen.

Vorzugsweise ist vorgesehen, dass die Zustandsbestimmungseinheit ein Ernährungszustandsindexmodul und ein Zustandsanalysemodul umfasst,

dass das Ernährungszustandsindexmodul zum Berechnen eines entsprechenden umfassenden Ernährungszustandsindex basierend auf Gesamtkonzentrationsdaten verschiedener Nährstoffe in dem Wasserkörper des Zielgewässers ausgebildet ist,

$$TLI = k_1 TLI_{chl-a} + k_2 TLI_{tp} + k_3 TLI_{tn} + k_4 TLI_{sd} + k_5 TLI_{codm-n}$$

dabei $TLI$ den umfassenden Ernährungszustandsindex darstellt, $TLI_{chl-a}$, $TLI_{tp}$, $TLI_{tn}$, $TLI_{sd}$ und $TLI_{codm-n}$ den Zustandsindex entsprechend Chlorophyll a, Phosphor, Stickstoff, Transparenz und Sauerstoffgehalt darstellen und $k_1, k_2, k_3, k_4, k_5$ den Exponentialkoeffizienten darstellt,

Dass das Zustandsanalysemodul zum Bestimmen des Nährstoffgrades des Zielgewässers gemäß dem Carlson-Ernährungszustandsindex ausgebildet ist. Vorzugsweise ist vorgesehen, dass das Wasserblüten-Frühwarnungsmodul eine Algendichtevorhersageeinheit und eine Wasserblütenwahrscheinlichkeitsvorhersageeinheit umfasst, dass die Algendichtevorhersageeinheit zum Konstruieren eines Algendichtevorhersagemodells durch einen maschinellen Lernalgorithmus ausgebildet ist, dass die Eingabevariable des Algendichtevorhersagemodells den Ernährungszustand des Wasserkörpers umfasst, dass die Ausgabevariable einen Algendichtevorhersagewert umfasst,

dass die Wasserblütenwahrscheinlichkeitsvorhersageeinheit zum Berechnen und Bestimmen der Wahrscheinlichkeit des Auftretens von Wasserblüten basierend auf dem Algendichtevorhersagewert ausgebildet ist.

Vorzugsweise ist vorgesehen, dass das System auch ein Echtzeit-Parameteroptimierungsmodul umfasst,

Dass das Echtzeit-Parameteroptimierungsmodul zum dynamischen Optimieren und Aktualisieren eines Identifikationseingabefaktors der Oberflächenzustandsidentifikationseinheit, eines Beziehungsparameters der Beziehungsfunktion zwischen der Oberflächenkonzentration und der Vertikalkonzentration und eines Modellkoeffizienten des Algendichtevorhersagemodells ausgebildet ist.

Gemäß dem zweiten Aspekt wird gemäß den Ausführungsbeispielen der vorliegenden Anmeldung ein Verfahren zur intelligenten Frühwarnung vor Wasserblüten basierend auf der Vorhersage des Ernährungszustands von Gewässern bereitgestellt, aufweisend:

Sammeln der Multiskaleninformation des Wasserkörpers in dem Zielgewässer, wobei die Multiskaleninformation des Wasserkörpers physikalische und chemische Information des Wasserkörpers, Oberflächeninformation des Wasserkörpers und Referenzhydrologieinformation umfasst,

Invertieren und Bestimmen der Oberflächennährstoffkonzentration basierend auf der Oberflächeninformation des Wasserkörpers,

Vorhersagen der Vertikalnährstoffkonzentration des Wasserkörpers in dem Zielgewässer basierend auf der Oberflächennährstoffkonzentration durch eine Beziehungsfunktion zwischen der Oberflächenkonzentration und der Vertikalkonzentration,

Bestimmen des Ernährungszustands des Wasserkörpers in dem Zielgewässer basierend auf der Oberflächennährstoffkonzentration und der Vertikalnährstoffkonzentration, wobei die Beziehungsfunktion zwischen der Oberflächenkonzentration und der Vertikalkonzentration gemäß der Multiskaleninformation des Wasserkörpers angepasst und bestimmt wird, Vorhersagen der Wahrscheinlichkeit des Auftretens von Wasserblüten gemäß dem Ernährungszustand des Wasserkörpers und Erzeugen der entsprechenden Warnrückkopplungsbenachrichtigungsinformation gemäß der Wahrscheinlichkeit des Auftretens von Wasserblüten, und

digitales Anzeigen der Visualisierung des Ernährungszustands des Wasserkörpers, der Wahrscheinlichkeit des Auftretens von Wasserblüten und der Warnrückkopplungsbenachrichtigungsinformation.

Es ist ersichtlich, dass gemäß den Ausführungsbeispielen der vorliegenden Anmeldung ein System und ein Verfahren zur intelligenten Frühwarnung vor Wasserblüten basierend auf der Vorhersage des Ernährungszustands von Gewässern bereitgestellt wird, aufweisend die folgenden vorteilhaften technischen Wirkungen:

(1) Das Multiskaleninformation-Erfassungsmodul ist zum Sammeln der Multiskaleninformation des Wasserkörpers in dem Zielgewässer ausgebildet und das Ernährungszustand-Erkennungsmodul ist zum Identifizieren der Oberflächennährstoffkonzentration basierend auf der Multiskaleninformation des Wasserkörpers und Vorhersagen der Vertikalnährstoffkonzentration ausgebildet, so dass die Oberflächennährstoffkonzentration mit der Vertikalnährstoffkonzentration kombiniert werden, um den Ernährungszustand des Wasserkörpers in den Zielgewässern zu analysieren und zu bestimmen. Ferner ist vorgesehen, dass basierend auf dem Ernährungszustand von Gewässern die Wahrscheinlichkeit des Auftretens von Wasserblüten vorhergesagt und bestimmt werden kann, um eine rechtzeitige Frühwarnung durchzuführen.

Wobei die Multiskaleninformation des Wasserkörpers physikalische und chemische Information des Wasserkörpers, Oberflächeninformation des Wasserkörpers und Referenzhydrologieinformation umfasst. Die Multiskaleninformation des Wasserkörpers ist als Datenbasis des Systems zur intelligenten Frühwarnung vor Wasserblüten basierend auf der Vorhersage des Ernährungszustands der Gewässer ausgebildet, um die Dateninformation umfassender zu machen, wodurch die Genauigkeit und Effektivität der Überwachungsergebnisse sichergestellt wird. Durch das Ernährungszustand-Erkennungsmodul wird zuerst die Oberflächennährstoffkonzentration des entsprechenden Wasserkörpers gemäß der Oberflächeninformation des Wasserkörpers invertiert und bestimmt. Dann wird auf der Grundlage der Beziehungsfunktion zwischen der Oberflächenkonzentration und der Vertikalkonzentration die Vertikalnährstoffkonzentration innerhalb des Wasserkörpers vorhergesagt und berechnet, so dass die Konzentrationsdaten der beiden Aspekte kombiniert werden, um die Zielgewässer als Ganzes zu analysieren. Auf diese Weise wird der Prozess von den Basisdaten bis zum endgültigen Überwachungsergebnis zerlegt, so dass die Nährstoffinformationen der Oberflächenschicht bzw. des Vertikalwassers des Wasserkörpers getrennt erhalten werden können. Die Prozessdaten sind umfassend, wodurch die Genauigkeit der Berechnung jedes Schritts im Prozess sichergestellt wird, wodurch sichergestellt wird, dass die Ergebnisse der Gesamtlösung genau und effektiv sind. Auf diese Weise kann die Vollständigkeit der Basisdaten und der Zwischenprozessinformationen sichergestellt werden, so dass der Ernährungszustand des Wasserkörpers in den Zielgewässern effizient und präzis identifiziert werden kann. Basierend auf dem genauen und präzisen Ernährungszustand des Wasserkörpers werden die Identifizierungsergebnisse vorhergesagt und die Wahrscheinlichkeit des Auftretens von Wasserblüten wird weiter vorhergesagt, so dass eine rechtzeitige und effektive Frühwarnung erreicht werden kann.

(2) Hydrodynamikdaten, Wassertemperaturdaten und Wasserqualitätsdaten werden durch die Unterwasser-Multifunktionsdetektionsvorrichtung gesammelt, dass der Echtzeitzustand des Wasserkörpers mit verschiedenen Detektionstiefen im Wasserkörper durch den Wasserfarbbildgeber fotografiert wird. Während des Detektionsprozesses wird der Wasserqualitätsüberwachungspunkt mit der Dämpfungsänderung der Turbulenzintensität des Wasserflusses mit variabler Frequenz abgetastet, so dass die Detektionstiefe und der Abstand zwischen den zwei Wasserfarbbildgebungsplatten entsprechend geändert werden. Unter der Dämpfungsänderung der Turbulenzintensität des Wasserflusses wird der Strahlungsstrahl abgetastet und abgebildet, um eine Bandkombination der Wasserqualität zu erzeugen. Auf diese Weise kann die Klarheit der Bildgebung sichergestellt werden, wodurch die Gültigkeit und Genauigkeit der gesammelten Wasserqualitätsdaten sichergestellt wird.

(3) Die fehlende Periode wird vorhergesagt und berechnet, so dass die Oberflächennährstoffkonzentration während der gesamten Periode erhalten werden kann. Auf der Ebene der Zeitkontinuität wird die Datenintegrität der Oberflächennährstoffkonzentration, die durch Invertieren und Identifizieren der Oberflächenzustandsidentifikationseinheit erhalten wird, verbessert. Basierend auf der Oberflächennährstoffkonzentration während der gesamten Periode kann eine zeitnahe Überwachung des Ernährungszustands des Zielgewässers erreicht werden, um das Eutrophierungsrisiko des Wasserkörpers zu verringern.

(4) Ein Algendichtevorhersagemodell wird durch den maschinellen Lernalgorithmus konstruiert, um den Algendichtewert des Zielgewässers vorherzusagen, so dass die Wahrscheinlichkeit des Auftretens von Wasserblüten basierend auf dem Algendichtewert genau vorhergesagt werden kann, wodurch eine rechtzeitige Frühwarnung für den Wasserblütenausbruch durchgeführt wird.

(5) Der Identifikationseingabefaktor der Oberflächenzustandsidentifikationseinheit, der Beziehungsparameter der Beziehungsfunktion zwischen der Oberflächenkonzentration und der Vertikalkonzentration und der Modellkoeffizient des Algendichtevorhersagemodells werden dynamisch optimiert und aktualisiert. Das Invertieren und Bestimmen der Oberflächennährstoffkonzentration, die Vorhersage der Vertikalnährstoffkonzentration und die Vorhersage der Wahrscheinlichkeit des Auftretens von Wasserblüten können eine hohe Genauigkeit aufrechterhalten, wodurch die Betriebsstabilität des Systems zur intelligenten Frühwarnung vor Wasserblüten basierend auf der Vorhersage des Ernährungszustands von Gewässern sichergestellt wird.

<u>Kurzbeschreibung der Zeichnungen</u>

Die Merkmale und Vorteile der vorliegenden Anmeldung werden durch Bezugnahme auf die Zeichnungen klarer verstanden. Die Zeichnungen sind schematisch und sollten nicht als Einschränkung der vorliegenden Anmeldung verstanden werden. Es zeigt:

Fig. 1 zeigt ein schematisches Diagramm eines Systems zur intelligenten Frühwarnung vor Wasserblüten basierend auf der Vorhersage des Ernährungszustands von Gewässern gemäß einem Ausführungsbeispiel der vorliegenden Anwendung;

Fig. 2 zeigt ein schematisches Diagramm eines Multiskaleninformation-Erfassungsmoduls in einem System zur intelligenten Frühwarnung vor Wasserblüten basierend auf der Vorhersage des Ernährungszustands von Gewässern gemäß einem Ausführungsbeispiel der vorliegenden Anwendung;

Fig. 3 zeigt ein schematisches Diagramm einer Struktur einer Unterwasser-Multifunktionsdetektionsvorrichtung in einem System zur intelligenten Frühwarnung vor Wasserblüten basierend auf der Vorhersage des Ernährungszustands von Gewässern gemäß einem Ausführungsbeispiel der vorliegenden Anwendung;

Fig. 4 zeigt ein schematisches Diagramm eines Ernährungszustand-Erkennungsmoduls in einem System zur intelligenten Frühwarnung vor Wasserblüten basierend auf der Vorhersage des Ernährungszustands von Gewässern gemäß einem Ausführungsbeispiel der vorliegenden Anwendung;

Fig. 5 zeigt ein schematisches Diagramm eines Wasserblüten-Frühwarnungsmoduls in einem System zur intelligenten Frühwarnung vor Wasserblüten basierend auf der Vorhersage des Ernährungszustands von Gewässern gemäß einem Ausführungsbeispiel der vorliegenden Anwendung;

Fig. 6 zeigt ein schematisches Diagramm eines Verfahrens zur intelligenten Frühwarnung vor Wasserblüten basierend auf der Vorhersage des Ernährungszustands von Gewässern gemäß einem Ausführungsbeispiel der vorliegenden Anwendung.

Beschreibung der bevorzugten Ausführungsbeispiele

Um den Zweck, die technische Lösung und die Vorteile der Ausführungsbeispiele der vorliegenden Anmeldung klarer zu machen, wird die technische Lösung in den Ausführungsbeispielen der vorliegenden Anmeldung im Folgenden in Verbindung mit den Zeichnungen in den Ausführungsbeispielen der vorliegenden Anmeldung klar und vollständig beschrieben. Offensichtlich sind die beschriebenen Ausführungsbeispiele einige Ausführungsbeispiele und nicht alle Ausführungsbeispiele der vorliegenden Anmeldung. Auf der Grundlage der Ausführungsbeispiele in der vorliegenden Anmeldung fallen alle anderen Ausführungsbeispiele, die der Fachmann ohne kreative Arbeit erhalten hat, in den Schutzbereich der vorliegenden Anmeldung.

In einigen verwandten Wasserkörperüberwachungstechnologien werden die meisten von ihnen durch Fernerkundungssatelliten, Drohnenfotografie oder manuelle Probenahmetests überwacht, so dass im Allgemeinen nur der Zustand von Oberflächenwasserkörpern identifiziert werden kann. Die Fernerkundungsüberwachung hat Zeitintervalle, in denen Überwachungsdaten fehlen. Die mangelnde Integrität der Überwachungsdaten und der komplexe Mechanismus des Auftretens von Wasserblüten erschweren eine umfassende Überwachung des Ernährungszustands von Wasserkörpern. Daher ist es unmöglich, den Ernährungszustand des Wasserkörpers präzis zu identifizieren, was die Praktikabilität der Lösung schlecht macht.

Angesichts der obigen Probleme wird gemäß den Ausführungsbeispielen der vorliegenden Anmeldung ein System und ein Verfahren zur intelligenten Frühwarnung vor Wasserblüten basierend auf der Vorhersage des Ernährungszustands von Gewässern bereitgestellt: Sammeln der Multiskaleninformation des Wasserkörpers in dem Zielgewässer; Bestimmen der Oberflächennährstoffkonzentration und der Vertikalnährstoffkonzentration des Wasserkörpers in dem Zielgewässer basierend auf Multiskaleninformation des Wasserkörpers; Bestimmen des gesamten Ernährungszustands des Wasserkörpers in dem Zielgewässer durch umfassende Analyse, so dass der Ernährungszustand des Wasserkörpers effizient und präzis identifiziert und bestimmt werden kann.

Im Folgenden werden die technischen Lösungen der vorliegenden Anmeldung in Verbindung mit spezifischen Ausführungsbeispielen beschrieben.

Auf der einen Seite wird gemäß den Ausführungsbeispielen der vorliegenden Anmeldung ein System zur Überwachung des Ernährungszustands von Gewässern bereitgestellt.

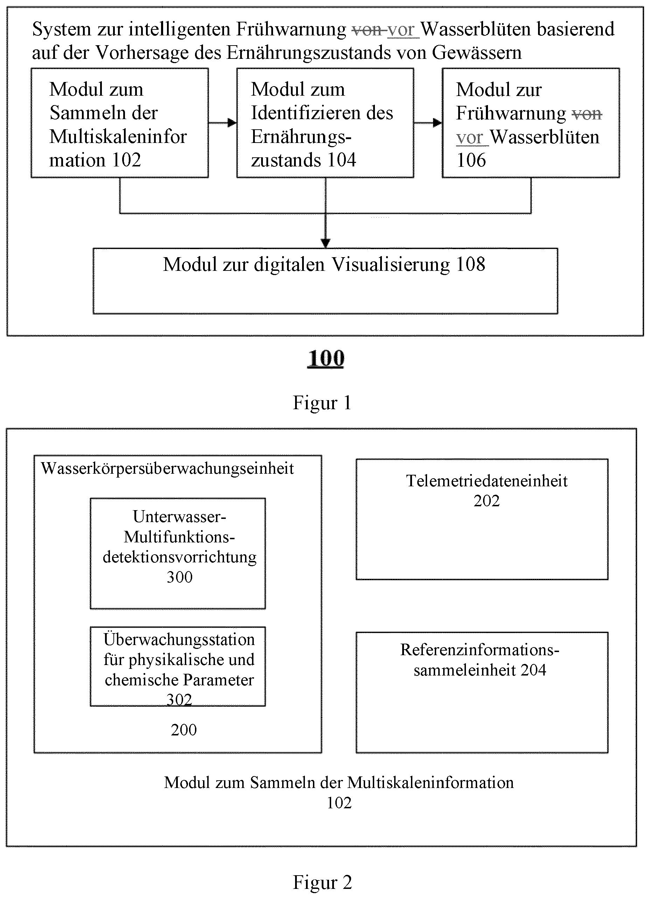

Gemäß einem oder mehreren optionalen Ausführungsbeispielen der vorliegenden Anmeldung wird ein System 100 zur intelligenten Frühwarnung vor Wasserblüten basierend auf der Vorhersage des Ernährungszustands von Gewässern bereitgestellt, wie in Fig. 1 gezeigt, umfassend:
Ein Multiskaleninformation-Erfassungsmodul 102 zum Sammeln der Multiskaleninformation des Wasserkörpers in dem Zielgewässer, wobei die Multiskaleninformation des Wasserkörpers physikalische und chemische Information des Wasserkörpers, Oberflächeninformation des Wasserkörpers und Referenzhydrologieinformation umfasst.

Die physikalischen und chemischen Informationen des Wasserkörpers können Informationen über die physikalischen Eigenschaften des Wasserkörpers in dem Zielgewässer, wie z. B. Strömungsgeschwindigkeit, Strömungsrichtung, Temperatur, Leitfähigkeit usw. und Informationen über die biochemischen Eigenschaften des Wasserkörpers in dem Zielgewässer, wie z. B. Algendichte, Stickstoffgehalt, Phosphorgehalt usw. umfassen, so dass die physikalischen und chemischen Informationen des Wasserkörpers den Ernährungszustand des Wasserkörpers seitlich widerspiegeln können.

Die Referenzhydrologieinformation kann historische Daten von relevanten Wasserstandsinformationen des Wasserkörpers in dem Zielgewässer umfassen, wie z. B. Wassermengeninformationen des Wasserspiegels, Topographie und Geomorphologie des Gewässers, meteorologische Umweltinformationen des Gewässers, Sedimentgehaltsinformationen, Hochwassersaisonzeitinformationen usw.

Ein Ernährungszustand-Erkennungsmodul 104 zum Invertieren und Bestimmen der Oberflächennährstoffkonzentration basierend auf der Oberflächeninformation des Wasserkörpers, zum Vorhersagen der Vertikalnährstoffkonzentration des Wasserkörpers in dem Zielgewässer basierend auf der Oberflächennährstoffkonzentration durch eine Beziehungsfunktion zwischen der Oberflächenkonzentration und der Vertikalkonzentration und zum Bestimmen des Ernährungszustands des Wasserkörpers in dem Zielgewässer basierend auf der Oberflächennährstoffkonzentration und der Vertikalnährstoffkonzentration.

Wobei die Beziehungsfunktion zwischen der Oberflächenkonzentration und der Vertikalkonzentration gemäß der Multiskaleninformation des Wasserkörpers angepasst und bestimmt wird.

In einigen optionalen Ausführungsbeispielen kann ein Trainingsdatensatz durch die Multiskaleninformation des Wasserkörpers durch maschinelles Lernen konstruiert werden, um die Beziehungsfunktion zwischen der Oberflächenkonzentration und der Vertikalkonzentration zu lernen und zu bestimmen, dass die verwendeten maschinellen Lernalgorithmen künstliche neuronale Netzwerke, Unterstützungsvektorregression, zufällige Wälder usw. umfassen, sind aber nicht darauf beschränkt.

Ein Wasserblüten-Frühwarnungsmodul 106 zum Vorhersagen der Wahrscheinlichkeit des Auftretens von Wasserblüten gemäß dem Ernährungszustand des Wasserkörpers und zum Erzeugen der entsprechenden Warnrückkopplungsbenachrichtigungsinformation gemäß der Wahrscheinlichkeit des Auftretens von Wasserblüten.

Ein Digitalvisualisierungsmodul 108 zum digitalen Anzeigen der Visualisierung der Oberflächennährstoffkonzentration, der Vertikalnährstoffkonzentration, des Ernährungszustands des Wasserkörpers, der Wahrscheinlichkeit des Auftretens von Wasserblüten und der Warnrückkopplungsbenachrichtigungsinformation.

Die visuelle Anzeige von Daten ist lebendiger, was einer dynamischen Überwachung in Echtzeit förderlich ist.

Das Multiskaleninformation-Erfassungsmodul im System zur intelligenten Frühwarnung vor Wasserblüten basierend auf der Vorhersage des Ernährungszustands von Gewässern ist zum Sammeln der Multiskaleninformation des Wasserkörpers in dem Zielgewässer ausgebildet und das Ernährungszustand-Erkennungsmodul ist zum Identifizieren der Oberflächennährstoffkonzentration basierend auf der Multiskaleninformation des Wasserkörpers und Vorhersagen der Vertikalnährstoffkonzentration ausgebildet, so dass die Oberflächennährstoffkonzentration mit der Vertikalnährstoffkonzentration kombiniert werden, um den Ernährungszustand des Wasserkörpers in den Zielgewässern zu analysieren und zu bestimmen.

Wobei die Multiskaleninformation des Wasserkörpers physikalische und chemische Information des Wasserkörpers, Oberflächeninformation des Wasserkörpers und Referenzhydrologieinformation umfasst. Die Multiskaleninformation des Wasserkörpers ist als Datenbasis des Systems zur intelligenten Frühwarnung vor Wasserblüten basierend auf der Vorhersage des Ernährungszustands der Gewässer ausgebildet, um die Dateninformation umfassender zu machen, wodurch die Genauigkeit und Effektivität der Überwachungsergebnisse sichergestellt wird. Durch das Ernährungszustand-Erkennungsmodul wird zuerst die Oberflächennährstoffkonzentration des entsprechenden Wasserkörpers gemäß der Oberflächeninformation des Wasserkörpers invertiert und bestimmt. Dann wird auf der Grundlage der Beziehungsfunktion zwischen der Oberflächenkonzentration und der Vertikalkonzentration die Vertikalnährstoffkonzentration innerhalb des Wasserkörpers vorhergesagt und berechnet, so dass die Konzentrationsdaten der beiden Aspekte kombiniert werden, um die Zielgewässer als Ganzes zu analysieren. Auf diese Weise wird der Prozess von den Basisdaten bis zum endgültigen Überwachungsergebnis zerlegt, so dass die Nährstoffinformationen der Oberflächenschicht bzw. des Vertikalwassers des Wasserkörpers getrennt erhalten werden können. Die Prozessdaten sind umfassend, wodurch die Genauigkeit der Berechnung jedes Schritts im Prozess sichergestellt wird, wodurch sichergestellt wird, dass die Ergebnisse der Gesamtlösung genau und effektiv sind. Auf diese Weise kann im System zur intelligenten Frühwarnung vor Wasserblüten basierend auf der Vorhersage des Ernährungszustands von Gewässern die Vollständigkeit der Basisdaten und der Zwischenprozessinformationen sichergestellt werden, so dass der Ernährungszustand des Wasserkörpers in den Zielgewässern effizient und präzis identifiziert werden kann.

Gemäß einem oder mehreren optionalen Ausführungsbeispielen der vorliegenden Anmeldung wird ein System 100 zur intelligenten Frühwarnung vor Wasserblüten basierend auf der Vorhersage des Ernährungszustands von Gewässern bereitgestellt, dass das Multiskaleninformation-Erfassungsmodul 102 eine Wasserkörpersüberwachungseinheit 200, eine Telemetriedateneinheit 202 und eine Referenzinformationssammeleinheit 204 umfasst, wie in Fig. 2 gezeigt.

Dass die Wasserkörpersüberwachungseinheit 200 zum Erhalten von Hydrodynamikdaten, Wassertemperaturdaten, Wasserqualitätsdaten und Gewässerwinddaten des Wasserkörpers in dem Zielgewässer ausgebildet ist, dass die physikalische und chemische Information des Wasserkörpers Hydrodynamikdaten, Wassertemperaturdaten, Wasserqualitätsdaten und Gewässerwinddaten des Wasserkörpers umfasst.

In einigen optionalen Ausführungsbeispielen kann die Wasserkörpersüberwachungseinheit 200 eine Unterwasser-Multifunktionsdetektionsvorrichtung 300 und eine Überwachungsstation 302 für physikalische und chemische Parameter umfassen, dass die Unterwasser-Multifunktionsdetektionsvorrichtung zum Erhalten von den Hydrodynamikdaten, den Wassertemperaturdaten und den Wasserqualitätsdaten ausgebildet ist, dass die Überwachungsstation für physikalische und chemische Parameter zum ergänzenden Überwachen der Wasserqualitätsdaten ausgebildet ist. Zum Beispiel können die Leitfähigkeit, der pH-Wert, der Kaliumpermanganatindex, der Sedimentgehaltsindex usw. des Wasserkörpers gesammelt werden.

Dass die Telemetriedateneinheit 202 zum Erhalten von Bilddaten in geringer Höhe und Fernerkundungsdaten in großer Höhe des Zielgewässers ausgebildet ist, dass die Oberflächeninformation des Wasserkörpers die Bilddaten in geringer Höhe und die Fernerkundungsdaten in großer Höhe umfasst,
Dass die Bilddaten in geringer Höhe durch Drohnenfotografie erhalten werden können, dass die Fernerkundungsdaten in großer Höhe durch hochauflösende Fernerkundungssatelliten gesammelt werden können.

Dass die Referenzinformationssammeleinheit 204 zum Sammeln der entsprechenden Hydrologie- und Hydrodynamikdaten, Gewässerumweltdaten, Topographie- und Geomorphologiedaten sowie Meteorologiedaten des Zielgewässers ausgebildet ist, dass die Referenzhydrologieinformation die Hydrologie- und Hydrodynamikdaten, die Gewässerumweltdaten, die Topographie- und Geomorphologiedaten sowie die Meteorologiedaten umfasst.

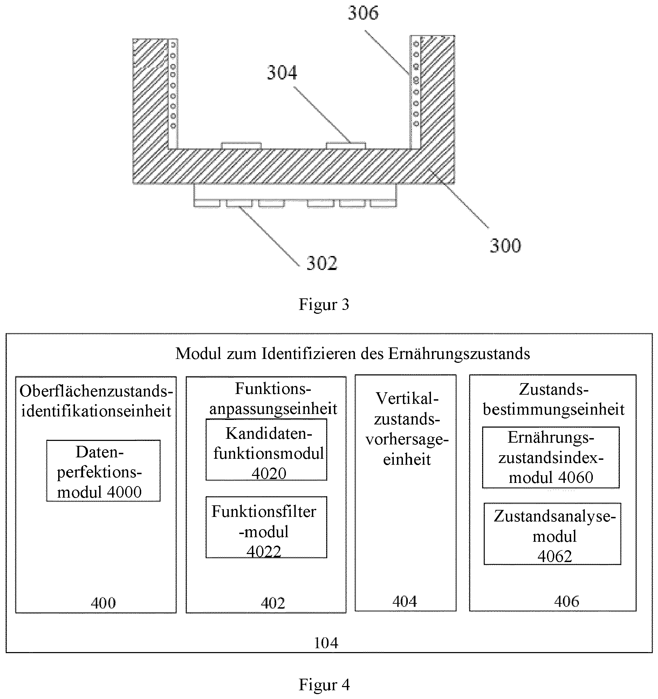

Gemäß einem oder mehreren optionalen Ausführungsbeispielen der vorliegenden Anmeldung wird ein System 100 zur intelligenten Frühwarnung vor Wasserblüten basierend auf der Vorhersage des Ernährungszustands von Gewässern bereitgestellt, dass die Unterwasser-Multifunktionsdetektionsvorrichtung 300 eine hydrodynamische Induktionssonde 302, einen Temperatursensor 304 und einen Wasserfarbbildgeber 306 umfasst, wie in Fig. 3 gezeigt.

Wobei die hydrodynamische Induktionssonde 302 am Unterteil der Unterwasser-Multifunktionsdetektionsvorrichtung 300 angeordnet ist, um hydrodynamische Faktoren mit unterschiedlichen Detektionstiefen zu detektieren und aufzuzeichnen.

Der Temperatursensor 304 kann in der Mitte der Unterwasser-Multifunktionsdetektionsvorrichtung 300 angeordnet sein, um Wassertemperaturdaten mit unterschiedlichen Detektionstiefen zu sammeln.

Dass der Wasserfarbbildgeber 306 zum Fotografieren an verschiedenen Detektionstiefen im Wasserkörper und zum Detektieren und Bestimmen von Sauerstoffauflösungsdaten, Chlorophylldaten, Algendichtedaten, Stickstoffdaten, Phosphordaten und Transparenzdaten im Wasserkörper ausgebildet ist, dass die Wasserqualitätsdaten Sauerstoffauflösungsdaten, Chlorophylldaten, Algendichtedaten, Stickstoffdaten, Phosphordaten und Transparenzdaten im Wasserkörper umfassen.

Wobei der Wasserfarbbildgeber 306 zwei symmetrisch verteilte Wasserfarbbildgebungsplatten 3060 umfasst.

Dass der Abstand zwischen den zwei Wasserfarbbildgebungsplatten 3060 gemäß der Detektionstiefe eingestellt wird:

$$w = \xi \sigma_i \frac{h}{e^\lambda + \eta}$$

dabei $w$ den Abstand zwischen den zwei Wasserfarbbildgebungsplatten darstellt, $\xi$ den Turbulenzkorrekturkoeffizienten darstellt, $\sigma_i$ die Turbulenzintensität des Wasserflusses am i Detektionspunkt darstellt, $h$ die Detektionstiefe entsprechend dem i Detektionspunkt darstellt, $\lambda$ den spektralen Auflösungsfaktor darstellt und $\eta$ den Korrekturparameter darstellt, Hydrodynamikdaten, Wassertemperaturdaten und Wasserqualitätsdaten werden im System 100 zur intelligenten Frühwarnung vor Wasserblüten basierend auf der Vorhersage des Ernährungszustands von Gewässern durch die Unterwasser-Multifunktionsdetektionsvorrichtung gesammelt, dass der Echtzeitzustand des Wasserkörpers mit verschiedenen Detektionstiefen im Wasserkörper durch den Wasserfarbbildgeber fotografiert wird. Während des Detektionsprozesses wird der Wasserqualitätsüberwachungspunkt mit der Dämpfungsänderung der Turbulenzintensität des Wasserflusses mit variabler Frequenz abgetastet, so dass die Detektionstiefe und der Abstand zwischen den zwei Wasserfarbbildgebungsplatten 3060 entsprechend geändert werden. Unter der Dämpfungsänderung der Turbulenzintensität des Wasserflusses wird der Strahlungsstrahl abgetastet und abgebildet, um eine Bandkombination der Wasserqualität zu erzeugen. Auf diese Weise kann die Klarheit der Bildgebung sichergestellt werden, wodurch die Gültigkeit und Genauigkeit der gesammelten Wasserqualitätsdaten sichergestellt wird.

Gemäß einem oder mehreren optionalen Ausführungsbeispielen der vorliegenden Anmeldung wird ein System 100 zur intelligenten Frühwarnung vor Wasserblüten basierend auf der Vorhersage des Ernährungszustands von Gewässern bereitgestellt, dass das Ernährungszustand-Erkennungsmodul 104 eine Oberflächenzustandsidentifikationseinheit 400, eine Funktionsanpassungseinheit 402, eine Vertikalzustandsvorhersageeinheit 404 und eine Zustandsbestimmungseinheit 406 umfasst, wie in Fig. 4 gezeigt.

Dass die Oberflächenzustandsidentifikationseinheit 400 zum Invertieren und Identifizieren des Ernährungszustands des Oberflächenwasserkörpers in dem Zielgewässer gemäß der Oberflächeninformation des Wasserkörpers ausgebildet ist, um die Oberflächennährstoffkonzentration zu bestimmen.

Dass die Oberflächenzustandsidentifikationseinheit 400 zum Invertieren und Identifizieren basierend auf einer Regressionsbeziehung zwischen dem Eingabeerkennungsfaktor und dem Ernährungszustand unter Verwendung der Bilddaten in geringer Höhe und Fernerkundungsdaten in großer Höhe des Zielgewässers als Eingabe, um die Oberflächennährstoffkonzentration zu bestimmen ausgebildet ist.

Dass die Funktionsanpassungseinheit 402 zum Konstruieren des Trainingsdatensatzes unter Verwendung der Multiskaleninformation des Wasserkörpers ausgebildet ist, dass das Training durch einen maschinellen Lernalgorithmus durchgeführt wird, um die Beziehungsfunktion zwischen der Oberflächenkonzentration und der Vertikalkonzentration anzupassen und zu bestimmen.

Dass die Beziehungsfunktion zwischen der Oberflächenkonzentration und der Vertikalkonzentration ausgebildet ist, um eine Beziehung zwischen der Nährstoffkonzentration in dem Oberflächenwasserkörper des Zielgewässers und der Nährstoffkonzentration in dem Vertikalwasserkörper des Zielgewässers zu beschreiben.

die Beziehungsfunktion zwischen der Oberflächenkonzentration und der Vertikalkonzentration:

$$F = \left( T, c_j^s, c_j^n, u, h, D_s \right)$$

dabei *F* die Beziehungsfunktion zwischen der Oberflächenkonzentration und der Vertikalkonzentration darstellt, T die Wasserkörperstemperatur darstellt, $c_i^s$ die Konzentration des *j* Nährstoffs in dem Oberflächenwasserkörper darstellt, $c_i^n$ die Konzentration des *j* Nährstoffs in dem Vertikalwasserkörper darstellt, *u*: die Strömungsgeschwindigkeit des Wasserflusses darstellt, *h* die Wassertiefe darstellt und $D_s$ den Komponentenausdehnungskoeffizienten darstellt,

In einigen optionalen Ausführungsbeispielen umfasst die Funktionsanpassungseinheit 402 ein Kandidatenfunktionsmodul 4020 und ein Funktionsfiltermodul 4022.

Dass das Kandidatenfunktionsmodul 4020 zum Durchführen eines Lerntrainings gemäß dem Trainingsdatensatz ausgebildet ist, um mehrere Kandidatenbeziehungsfunktionen zu bestimmen.

Dass das Funktionsfiltermodul 4022 zum Berechnen und Bestimmen eines Bestimmungskoeffizienten mehrerer Kandidatenbeziehungsfunktionen ausgebildet ist, um eine optimale Funktion als die Beziehungsfunktion zwischen der Oberflächenkonzentration und der Vertikalkonzentration gemäß dem Bestimmungskoeffizienten auszuwählen.

Das Funktionsfiltermodul 4022 kann die Vorhersagegenauigkeit mehrerer Kandidatenbeziehungsfunktionen durch einen mittleren quadratischen Fehler, einen relativen mittleren quadratischen Fehler, einen relativen Fehler, einen umfassenden Fehler und einen Bestimmungskoeffizienten : $R^2$ messen, wodurch die optimale Funktion ausgewählt wird.

$$\text{RMSE} = \sqrt{\frac{\sum_{i=1}^{n} \hat{y}_i - y_i^2}{n}}$$

Mittlerer quadratischer Fehler

$$\text{rRMSE} = \frac{\text{RMSE}}{\bar{y}}$$

Relativer mittlerer quadratischer Fehler

$$\text{ARE} = \sum_{i=1}^{n} \frac{\left|\frac{\hat{y}_i - y_i}{y_i}\right|}{n} \times 100\%$$

Relativer Fehler

$$\text{CE} = \frac{CE_{-c} + CE_{-v}}{2} = \frac{\text{rRMSE}_{-c} + \text{ARE}_{-c} + \text{rRMSE}_{-v} + \text{ARE}_{-v}}{4}$$

Umfassender Fehler

$$\text{R}^2 = \frac{SSR}{SST} = 1 - \frac{SSE}{SST}$$

Bestimmungskoeffizient:                    (davon: SST = SSR + SSE)

Dabei $y_i$ der gemessene Wert der Wasserqualitätsparameter am Probenahmepunkt *i*: darstellt, $\bar{y}$ der Durchschnitt der gemessenen Werte der Wasserqualitätsparameter darstellt, $\hat{y}_i$ der Inversionswert der Wasserqualitätsparameter am Probenahmepunkt *i*: darstellt, n die Beziehungsfunktion oder die Anzahl der entsprechenden Abtastpunkte zur Verifizierung darstellt, $CE_{-c}$, $CE_{-v}$ der umfassende Fehler der Beziehungsfunktion und der Verifizierung darstellt, SST die Summe der Quadrate der Gesamtdispersion darstellt, SSR die Summe der Quadrate der Regression darstellt und SSE die Summe der Quadrate der Residuen darstellt.

Dass die Vertikalzustandsvorhersageeinheit 404 zum Vorhersagen und Berechnen der Oberflächennährstoffkonzentration als Eingabe basierend auf der Beziehungsfunktion zwischen der Oberflächenkonzentration und der Vertikalkonzentration und Bestimmen der Vertikalnährstoffkonzentration basierend auf der Ausgabe der Beziehungsfunktion zwischen der Oberflächenkonzentration und der Vertikalkonzentration ausgebildet ist.

Dass die Zustandsbestimmungseinheit 406 zum Analysieren und Bestimmen des Ernährungszustands des Wasserkörpers in dem Zielgewässer basierend auf der Oberflächennährstoffkonzentration und der Vertikalnährstoffkonzentration ausgebildet ist, dass die Zustandsbestimmungseinheit 406 Daten der Oberflächennährstoffkonzentration und der Vertikalnährstoffkonzentration des Wasserkörpers in dem Zielgewässer kombinieren kann, um eine Gesamtanalyse des Wasserkörpers durchzuführen.

Dass die Zustandsbestimmungseinheit 406 ein Ernährungszustandsindexmodul 4060 und ein Zustandsanalysemodul 4062 umfasst.

Dass das Ernährungszustandsindexmodul 4060 zum Berechnen eines entsprechenden umfassenden Ernährungszustandsindex basierend auf Gesamtkonzentrationsdaten verschiedener Nährstoffe in dem Wasserkörper des Zielgewässers ausgebildet ist.

$$TLI = k_1 TLI_{chl-a} + k_2 TLI_{tp} + k_3 TLI_{tn} + k_4 TLI_{sd} + k_5 TLI_{codm-n}$$

dabei $TLI$ den umfassenden Ernährungszustandsindex darstellt, $TLI_{chl-a}, TLI_{tp}, TLI_{tn}$, $TLI_{sd}$ und $TLI_{codm-n}$ den Zustandsindex entsprechend Chlorophyll a, Phosphor, Stickstoff, Transparenz und Sauerstoffgehalt darstellen und $k_1, k_2, k_3, k_4, k_5$ den Exponentialkoeffizienten darstellt,

Dass das Zustandsanalysemodul 4062 zum Bestimmen des Nährstoffgrades des Zielgewässers gemäß dem Carlson-Ernährungszustandsindex ausgebildet ist.

Darunter ist TLI < 30 ein oligotropher Grad, 30 ≤ TLI ≤ 50 ein mesotropher Grad, 50 < TLI ≤ 60 ein milder eutropher Grad, 60 < TLI ≤ 70 ein mäßiger eutropher Grad, TLI > 70 ein schwerer eutropher Grad.

Gemäß einem oder mehreren optionalen Ausführungsbeispielen der vorliegenden Anmeldung wird ein System 100 zur intelligenten Frühwarnung vor Wasserblüten basierend auf der Vorhersage des Ernährungszustands von Gewässern bereitgestellt, dass die Oberflächenzustandsidentifikationseinheit 400 ein Datenverbesserungsmodul 4000 umfasst, wie in Fig. 4 gezeigt.

Dass das Datenverbesserungsmodul 4000 zum Vorhersagen und Berechnen der mit dem fehlenden Periode der Oberflächeninformation des Wasserkörpers entsprechenden Oberflächennährstoffkonzentration bei der Bestimmung der Oberflächennährstoffkonzentration ausgebildet ist,

$$c_{t+\Delta t} = \psi \left( \frac{u}{\sqrt{S} D_s} \Delta t \right) c_t$$

dabei $c_{t+\Delta t}$ die Oberflächennährstoffkonzentration zum Zeitpunkt $t + \Delta t$| darstellt, $c_t$ die Oberflächennährstoffkonzentration zum Zeitpunkt $t$ darstellt, $\Delta t$ die fehlende Zeit darstellt, $\psi$ den Korrekturkoeffizienten der Nährstoffkonzentration darstellt und $S$ die Oberflächenfläche des Zielgewässers darstellt,

In Anbetracht der Tatsache, dass die von der Telemetriedateneinheit gesammelten Bilddaten in geringer Höhe und Fernerkundungsdaten in großer Höhe aufgrund des Überwachungszeitintervalls einen Datenverlust aufweisen können, ist das Datenverbesserungsmodul 4000 für die fehlende Periode zur Vorhersage und Berechnung ausgebildet, so dass die Oberflächennährstoffkonzentration während der gesamten Periode erhalten werden kann. Auf der Ebene der Zeitkontinuität wird die Datenintegrität der Oberflächennährstoffkonzentration, die durch Invertieren und Identifizieren der Oberflächenzustandsidentifikationseinheit erhalten wird, verbessert. Basierend auf der Oberflächennährstoffkonzentration während der gesamten Periode kann eine zeitnahe Überwachung des Ernährungszustands des Zielgewässers durch das Ernährungszustand-Erkennungsmodul erreicht werden, um das Eutrophierungsrisiko des Wasserkörpers zu verringern.

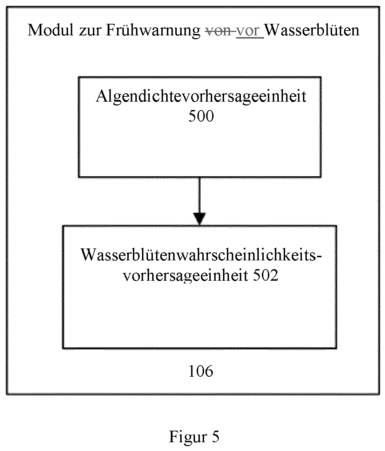

Gemäß einem oder mehreren optionalen Ausführungsbeispielen der vorliegenden Anmeldung wird ein System 100 zur intelligenten Frühwarnung vor Wasserblüten basierend auf der Vorhersage des Ernährungszustands von Gewässern bereitgestellt, dass das Wasserblüten-Frühwarnungsmodul 106 eine Algendichtevorhersageeinheit 500 und eine Wasserblütenwahrscheinlichkeitsvorhersageeinheit 502 umfasst, wie in Fig. 5 gezeigt.

Dass die Algendichtevorhersageeinheit 500 zum Konstruieren eines Algendichtevorhersagemodells durch einen maschinellen Lernalgorithmus ausgebildet ist, dass die Eingabevariable des Algendichtevorhersagemodells den Ernährungszustand des Wasserkörpers umfasst, dass die Ausgabevariable einen Algendichtevorhersagewert umfasst.

Dass die Algendichtevorhersageeinheit 500 zum Erlernen der Korrelation zwischen der Algendichte dominanter Algen und Umwelteinflussfaktoren in den Zielgewässern durch Konstruieren des Algendichtevorhersagemodells ausgebildet ist, wobei die Umwelteinflussfaktoren den Ernährungszustand des Wasserkörpers und Bedingungen wie Nährstoffe, Meteorologie und Hydrodynamik umfassen, so dass das Algendichtevorhersagemodell die Algendichte in dem Zielgewässer gemäß den aktuellen Umwelteinflussfaktoren genau vorhersagen kann.

Dass die Wasserblütenwahrscheinlichkeitsvorhersageeinheit 502 zum Berechnen und Bestimmen der Wahrscheinlichkeit des Auftretens von Wasserblüten basierend auf dem Algendichtevorhersagewert ausgebildet ist.

In einigen optionalen Ausführungsbeispielen kann der Algendichtewert durch einen Wert von Chlorophyll a (Chl-a) dargestellt werden, so dass der Vorhersagewert von Chl-a in dem Zielgewässer durch das Algendichtevorhersagemodell bestimmt wird. Dann ist der Vorhersagewert von Chl-a als Kriterium für das Auftreten von Wasserblüten ausgebildet. Wenn zum Beispiel sein Gehalt höher als 30 ist, kann die Möglichkeit eines Wasserblütenausbruchs bestimmt werden.

Das System zur intelligenten Frühwarnung vor Wasserblüten basierend auf der Vorhersage des Ernährungszustands von Gewässern führt auch eine Frühwarnung vor Wasserblüten basierend auf dem Ernährungszustand durch, nachdem der Ernährungszustand des Zielgewässers bestimmt wurde. Ein Algendichtevorhersagemodell wird durch den maschinellen Lernalgorithmus konstruiert, um den Algendichtewert des Zielgewässers vorherzusagen, wodurch die Wahrscheinlichkeit des Auftretens von Wasserblüten bestimmt wird und eine rechtzeitige Frühwarnung vor Wasserblütenausbrüchen durchgeführt werden kann.

Gemäß einem oder mehreren optionalen Ausführungsbeispielen der vorliegenden Anmeldung wird ein System 100 zur intelligenten Frühwarnung vor Wasserblüten basierend auf der Vorhersage des Ernährungszustands von Gewässern bereitgestellt, dass das System auch ein Echtzeit-Parameteroptimierungsmodul umfasst, dass das Echtzeit-Parameteroptimierungsmodul zum dynamischen Optimieren und Aktualisieren eines Identifikationseingabefaktors der Oberflächenzustandsidentifikationseinheit, eines Beziehungsparameters der Beziehungsfunktion zwischen der Oberflächenkonzentration und der Vertikalkonzentration und eines Modellkoeffizienten des Algendichtevorhersagemodells ausgebildet ist.

Für den Identifikationseingabefaktor filtert das Echtzeit-Parameteroptimierungsmodul Parameter, die einen großen Einfluss auf das Modell haben, durch einen Index, der sich auf den Ernährungszustand des Wasserkörpers bezieht, der durch eine Fernerkundungsbandkombination erhalten wird, und kombiniert mit einem Empfindlichkeitsanalysealgorithmus, um den repräsentativsten Spektralbereich, das Band, die Bandkombination und dergleichen zum Invertieren von Wasserumgebungsparametern zu bestimmen. Der Fernerkundungswasserkörperindex wird durch Bandverhältnis, Differenzierung erster Ordnung, Exponentialalgorithmus, Dreiband- und Vierbandalgorithmus berechnet. Korrelationsanalyse und Kollinearitätsdiagnose werden gemäß der Anzahl der gemessenen Probenpunkte durchgeführt, um Probenpunkte mit schlechter Korrelation und hoher linearer Beziehung zu eliminieren. Durch die Optimierung der Parameter wird schließlich die Regressionsbeziehung zwischen Fernerkundungsfaktoren und Ernährungszustand erhalten. Der ausgewählte Fernerkundungswasserkörperfaktor ist als der Identifikationseingabefaktor der Oberflächenzustandsidentifikationseinheit ausgebildet. Der Beziehungsparameter der Beziehungsfunktion zwischen der Oberflächenkonzentration und der Vertikalkonzentration und der Modellkoeffizient des Algendichtevorhersagemodells werden durch das Echtzeit-Parameteroptimierungsmodul dynamisch optimiert und aktualisiert. Das Invertieren und Bestimmen der Oberflächennährstoffkonzentration, die Vorhersage der Vertikalnährstoffkonzentration und die Vorhersage der Wahrscheinlichkeit des Auftretens von Wasserblüten können eine hohe Genauigkeit aufrechterhalten, wodurch die Betriebsstabilität des Systems zur intelligenten Frühwarnung vor Wasserblüten basierend auf der Vorhersage des Ernährungszustands von Gewässern sichergestellt wird.

System, Modul, Einheit oder Modul, das in dem obigen Ausführungsbeispiel veranschaulicht ist, kann insbesondere durch einen Computerchip oder eine Entität oder durch ein Produkt mit einer bestimmten Funktion implementiert werden. Ein typisches Implementierungsgerät ist ein Computer. Insbesondere kann ein Computer beispielsweise ein Personalcomputer, ein Laptop, ein Mobiltelefon, ein Kameratelefon, ein Smartphone, ein persönlicher digitaler Assistent, ein Media-Player, ein Navigationsgerät, ein E-Mail-Gerät, eine Spielkonsole, ein Tablet, ein tragbares Gerät oder eine Kombination eines dieser Geräte sein.

Zur Vereinfachung der Beschreibung werden bei der Beschreibung der obigen Vorrichtung die Funktionen in verschiedene Einheiten unterteilt und entsprechend beschrieben. Natürlich können beim Implementieren der vorliegenden Anmeldung die Funktionen jeder Einheit in derselben oder mehreren Software und/oder Hardware implementiert werden.

Basierend auf dem gleichen Erfindungszweck wird gemäß den Ausführungsbeispielen der vorliegenden Anmeldung auch ein Verfahren zur intelligenten Frühwarnung vor Wasserblüten basierend auf der Vorhersage des Ernährungszustands von Gewässern bereitgestellt.

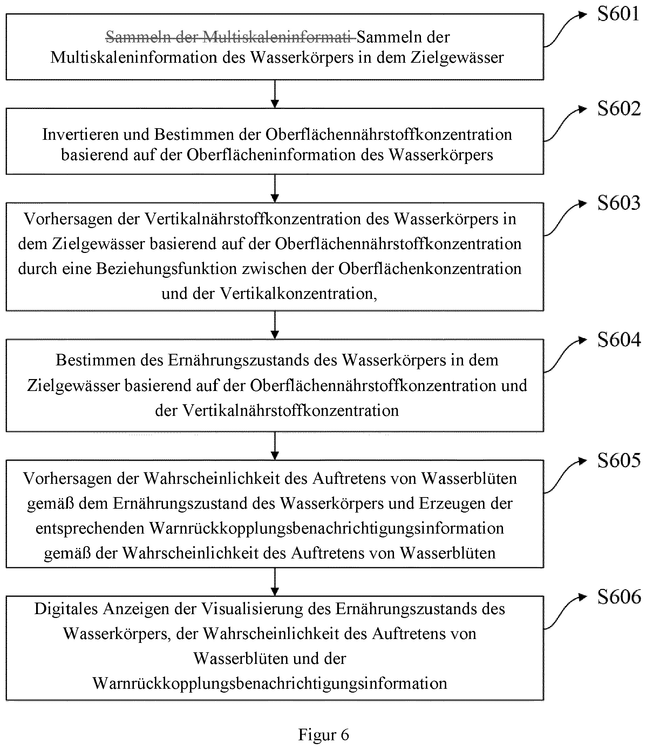

Gemäß einem oder mehreren optionalen Ausführungsbeispielen der vorliegenden Anmeldung wird ein Verfahren zur intelligenten Frühwarnung vor Wasserblüten basierend auf der Vorhersage des Ernährungszustands von Gewässern bereitgestellt, wie in Fig. 6 gezeigt, umfassend:

S601: Sammeln der Multiskaleninformation des Wasserkörpers in dem Zielgewässer, wobei die Multiskaleninformation des Wasserkörpers physikalische und chemische Information des Wasserkörpers, Oberflächeninformation des Wasserkörpers und Referenzhydrologieinformation umfasst.

S602: Invertieren und Bestimmen der Oberflächennährstoffkonzentration basierend auf der Oberflächeninformation des Wasserkörpers.

S603: Vorhersagen der Vertikalnährstoffkonzentration des Wasserkörpers in dem Zielgewässer basierend auf der Oberflächennährstoffkonzentration durch eine Beziehungsfunktion zwischen der Oberflächenkonzentration und der Vertikalkonzentration, wobei die Beziehungsfunktion zwischen der Oberflächenkonzentration und der Vertikalkonzentration gemäß der Multiskaleninformation des Wasserkörpers angepasst und bestimmt wird.

S604: Bestimmen des Ernährungszustands des Wasserkörpers in dem Zielgewässer basierend auf der Oberflächennährstoffkonzentration und der Vertikalnährstoffkonzentration.

S605: Vorhersagen der Wahrscheinlichkeit des Auftretens von Wasserblüten gemäß dem Ernährungszustand des Wasserkörpers und Erzeugen der entsprechenden Warnrückkopplungsbenachrichtigungsinformation gemäß der Wahrscheinlichkeit des Auftretens von Wasserblüten.
S606: Digitales Anzeigen der Visualisierung des Ernährungszustands des Wasserkörpers, der Wahrscheinlichkeit des Auftretens von Wasserblüten und der Warnrückkopplungsbenachrichtigungsinformation.

Am Beispiel eines empfindlichen Gewässers werden zur aktiven Regulierung und Bewirtschaftung der Ernährungszustand typischer empfindlicher Gewässer durch das System und Verfahren zur intelligenten Frühwarnung vor Wasserblüten basierend auf der Vorhersage des Ernährungszustands von Gewässern auf der Grundlage der technischen Lösung der vorliegenden Anmeldung identifiziert und die intelligente Frühwarnung vor Wasserblüten durchgeführt. Basierend auf den fünf Indexdaten chl-a, TP, TN, SD und CODMn wird der umfassende Ernährungszustandsindex TLI berechnet und identifiziert. Das Nährstoffgrade des Sees wird durch eine Reihe von aufeinanderfolgenden Zahlen von 0 bis 100 eingestuft: Darunter ist TLI < 30 ein oligotropher Grad, $30 \leq TLI \leq 50$ ein mesotropher Grad, $50 < TLI \leq 60$ ein milder eutropher Grad, $60 < TLI \leq 70$ ein mäßiger eutropher Grad, TLI > 70 ein schwerer eutropher Grad. Je höher der TLI-Indexwert im gleichen Ernährungszustand ist, desto schwerer ist der Ernährungsgrad des Sees. Am Beispiel der gemessenen Daten von 2001 bis 2021 liegen die meisten umfassenden Ernährungszustandsindizes, die in verschiedenen Jahren berechnet wurden, zwischen 50 und 60, was ein milder Eutrophierungsgrad ist. Unter ihnen erreichte der Maximalwert des umfassenden Ernährungszustandsindex im Jahr 2007 68,794, was ein mäßiger Eutrophierungsgrad ist. Der Maximalwert des umfassenden Ernährungszustandsindex im Jahr 2009 betrug 69,090, was ein mesotropher Eutrophierungsgrad ist. Unter Berücksichtigung der Korrelation zwischen der Konzentration von Chlorophyll a und anderen Faktoren wurden die Diskriminanzbedingungen für die Beeinflussung des Faktorsegmentierungswerts und die Auslösung des Wasserblütenausbruchs als Silicium-Phosphor-Verhältnis SiP < 72, Sauerstoffauflösung DO 9 mg/l, Transparenz SD < 92 cm, Silicium-Stickstoff-Verhältnis SiN < 5,7 und Gesamtphosphor TP 0,13 mg/l erhalten. Entsprechend dem Ernährungszustand des Wasserkörpers beträgt die Wahrscheinlichkeit des endgültigen Wasserblütenausbruchs 47,6%, was die Möglichkeit des Auftretens von Wasserblüten birgt. In diesem Fall werden entsprechende Warnrückkopplungsbenachrichtigungsinformationen erzeugt, so dass das zuständige Personal daran erinnert wird, in das Gewässer einzugreifen, um die Möglichkeit eines Wasserblütenausbruchs zu verringern. Die folgende Tabelle 1 ist eine Verifikationstabelle für die Ergebnisse und tatsächlichen Bedingungen der Überwachung und Identifizierung des Ernährungszustands von Gewässern durch die technische Lösung der vorliegenden Anmeldung.

Tabelle 1 Verifizierung der Erkennungsergebnisse des Multiskalen-Erkennungsmoduls

| Zeit | Stelle | Messwert TSI | Klassifizierung des Ernährungszustands | Vorhersagewert TSI | Klassifizierung des Ernährungszustands | Klassifizierungsfehler | Relativer Fehler % |
|---|---|---|---|---|---|---|---|
| 2007. 04.24 | XX00 | 49,794 | mesotroph | 47,971 | mesotroph | 1 | 3,800 |
| | XX01 | 55,306 | mild | 51,975 | mild | 1 | 6,408 |
| | XX02 | 52,516 | mild | 51,492 | mild | 1 | 1,988 |
| | XX03 | 43,748 | mesotroph | 45,424 | mesotroph | 1 | 3,689 |
| | XX04 | 43,096 | mesotroph | 45,987 | mesotroph | 1 | 6,287 |
| | XX05 | 40,585 | mesotroph | 37,445 | mesotroph | 1 | 8,385 |
| | XX06 | 40,648 | mesotroph | 36,611 | mesotroph | 1 | 11,027 |
| | XX07 | 42,677 | mesotroph | 37,116 | mesotroph | 1 | 14,984 |
| | XX08 | 33,390 | mesotroph | 36,763 | mesotroph | 1 | 9,173 |
| | XX09 | 33,244 | mesotroph | 39,792 | mesotroph | 1 | 16,457 |
| | XX10 | 27,122 | oligotroph | 41,772 | mesotroph | 0 | 35,072 |
| 2009 04.13 | XX00 | 34,831 | mesotroph | 44,317 | mesotroph | 1 | 21,405 |
| | XX01 | 47,538 | mesotroph | 47,654 | mesotroph | 1 | 0,245 |
| | XX02 | 52,065 | mild | 50,927 | mild | 1 | 2,235 |
| | XX03 | 50,184 | mild | 42,226 | mesotroph | 0 | 18,846 |
| | XX04 | 40,378 | mesotroph | 43,809 | mesotroph | 1 | 7,831 |
| | XX05 | 40,378 | mesotroph | 42,580 | mesotroph | 1 | 5,169 |
| | XX06 | 52,782 | mild | 56,879 | mild | 1 | 7,204 |
| | XX07 | 57,262 | mild | 55,461 | mild | 1 | 3,246 |
| | XX08 | 61,302 | mäßig | 59,887 | mild | 0 | 2,362 |
| | XX09 | 62,404 | mäßig | 63,138 | mäßig | 1 | 1,162 |
| | XX10 | 69,090 | mäßig | 59,265 | mild | 0 | 16,578 |
| | XXYT | 54,830 | mild | 57,102 | mild | 1 | 3,979 |

(fortgesetzt)

| Zeit | Stelle | Messwert TSI | Klassifizierung des Ernährungszustands | Vorhersagewert TSI | Klassifizierung des Ernährungszustands | Klassifizierungsfehler | Relativer Fehler % |
|---|---|---|---|---|---|---|---|
| | XX00 | 48,230 | mesotroph | 50,008 | mild | 0 | 3,554 |
| | XX01 | 51,441 | mild | 52,170 | mild | 1 | 1,398 |
| | XX02 | 57,897 | mild | 52,685 | mild | 1 | 9,893 |
| | XX03 | 55,808 | mild | 55,994 | mild | 1 | 0,333 |
| | XX04 | 61,271 | mäßig | 59,221 | mild | 0 | 3,462 |
| 2007 09.15 | XX05 | 66,210 | mäßig | 62,036 | mäßig | 1 | 6,727 |
| | XX06 | 62,462 | mäßig | 60,047 | mäßig | 1 | 4,022 |
| | XX07 | 60,235 | mäßig | 60,242 | mäßig | 1 | 0,012 |
| | XX08 | 60,877 | mäßig | 62,601 | mäßig | 1 | 2,753 |
| | XX09 | 68,794 | mäßig | 68,765 | mäßig | 1 | 0,043 |
| | XX10 | 41,585 | mesotroph | 57,362 | mild | 0 | 27,504 |
| Summe der relativen Fehler des Trainingssatzes | | | | | | | 7,860 |
| 2010. 10.05 | XX01 | 58,906 | 3 | 55,485 | mild | 1 | 6,166 |
| | XX04 | 67,112 | 4 | 59,854 | mild | 0 | 12,125 |
| | XX06 | 67,166 | 4 | 66,829 | mäßig | 1 | 0,504 |
| | XX09 | 58,124 | 3 | 66,396 | mäßig | 0 | 12,458 |
| Summe der relativen Fehler des Testsatzes | | | | | | | 7,813 |

Durch das System und Verfahren zur intelligenten Frühwarnung vor Wasserblüten basierend auf der Vorhersage des Ernährungszustands von Gewässern auf der Grundlage der technischen Lösung der vorliegenden Anmeldung beträgt der durchschnittliche Fehler der Vorhersage der Ernährung der Zielgewässer nur etwa 7,8%. Es ist ersichtlich, dass das System und Verfahren zur intelligenten Frühwarnung vor Wasserblüten basierend auf der Vorhersage des Ernährungszustands von Gewässern auf der Grundlage der technischen Lösung der vorliegenden Anmeldung eine effiziente und präzise Vorhersage und Identifizierung des Ernährungszustands der Wasserkörper in den Zielgewässern erreichen können, so dass die Frühwarnung vor Wasserblüten rechtzeitig und effektiv durchgeführt werden kann.

Es sei darauf hingewiesen, dass ein Verfahren gemäß einem Ausführungsbeispiel der vorliegenden Anmeldung von einem einzelnen Gerät, z. B. einem Computer oder Server, durchgeführt werden kann. Das Verfahren gemäß dem vorliegenden Ausführungsbeispiel kann auch auf ein verteiltes Szenario angewendet werden, das durch Zusammenwirken mehrerer Geräte abgeschlossen wird. In einem solchen verteilten Szenario kann eines der mehreren Geräte nur einen oder mehrere Schritte in einem Verfahren gemäß einem Ausführungsbeispiel der vorliegenden Anmeldung ausführen, so dass die mehreren Geräte miteinander interagieren, um das Verfahren abzuschließen.

Es sei darauf hingewiesen, dass einige Ausführungsbeispiele der vorliegenden Anmeldung oben beschrieben sind. Andere Ausführungsbeispiele liegen im Rahmen der beigefügten Ansprüche. In einigen Fällen können die in den Ansprüchen aufgezeichneten Aktionen oder Schritte in einer anderen Reihenfolge als in dem obigen Ausführungsbeispiel ausgeführt werden und können immer noch das gewünschte Ergebnis erzielen. Darüber hinaus ist der in den Zeichnungen dargestellte Prozess nicht notwendigerweise in einer bestimmten Reihenfolge oder kontinuierlichen Reihenfolge dargestellt, um das gewünschte Ergebnis zu erzielen. In einigen Ausführungsformen sind Multitasking und Parallelverarbeitung ebenfalls möglich oder können vorteilhaft sein.

Der gewöhnliche Fachmann sollte verstehen, dass die Erörterung einer der oben genannten Ausführungsbeispiele nur beispielhaft ist und nicht implizieren soll, dass der Umfang der vorliegenden Anmeldung (einschließlich der Ansprüche) auf diese Beispiele beschränkt ist; dass die obigen Ausführungsbeispiele oder die technischen Merkmale in verschiedenen Ausführungsbeispielen auch unter der Idee der vorliegenden Anmeldung kombiniert werden können. Die Schritte können in beliebiger Reihenfolge implementiert werden. Es gibt viele andere Änderungen in verschiedenen Aspekten der oben genannten Ausführungsbeispiele der vorliegenden Anmeldung, die nicht im Detail angegeben sind, um sie zu präzisieren.

Um die Beschreibung und Diskussion zu vereinfachen und die Ausführungsbeispiele der vorliegenden Anmeldung nicht schwer zu verstehen, kann außerdem eine bekannte Stromversorgung/Erdungsverbindung mit einem integrierten Schaltungschip (IC) und anderen Komponenten in den bereitgestellten Zeichnungen gezeigt oder nicht gezeigt werden. Ferner kann die Vorrichtung in Form eines Blockdiagramms gezeigt werden, um zu vermeiden, dass die Ausführungsbeispiele der vorliegenden Anmeldung schwer zu verstehen sind. Dies berücksichtigt auch die Tatsache, dass die Details der Ausführungsformen dieser Blockdiagrammvorrichtungen in hohem Maße von der Plattform abhängen, auf der die Ausführungsbeispiele der vorliegenden Anmeldung implementiert werden sollen (d.h. diese Details sollten vollständig im Rahmen des Verständnisses des Fachmanns liegen). In Fällen, in denen spezifische Details (z. B. Schaltungen) zur Beschreibung beispielhafter Ausführungsbeispiele der vorliegenden Anmeldung ausgearbeitet werden, kann der Fachmann die Ausführungsbeispiele der vorliegenden Anmeldung ohne diese spezifischen Details oder ohne Änderungen dieser spezifischen Details leicht ausführen. Daher sollten diese Beschreibungen als illustrativ und nicht restriktiv angesehen werden.

Zum Beispiel können andere Speicherarchitekturen (z. B. dynamischer RAM (DRAM)) für die diskutierten Ausführungsbeispiele verwendet werden.

**Patentansprüche**

1.  System (100) zur intelligenten Frühwarnung vor Wasserblüten basierend auf der Vorhersage des Ernährungszustands von Gewässern, **dadurch gekennzeichnet, dass** das System die folgenden Module umfasst:

    ein Multiskaleninformation-Erfassungsmodul (102) zum Sammeln der Multiskaleninformation des Wasserkörpers in dem Zielgewässer, wobei die Multiskaleninformation des Wasserkörpers physikalische und chemische Information des Wasserkörpers, Oberflächeninformation des Wasserkörpers und Referenzhydrologieinformation umfasst,
    ein Ernährungszustand-Erkennungsmodul (104) zum Invertieren und Bestimmen der Oberflächennährstoffkonzentration basierend auf der Oberflächeninformation des Wasserkörpers, zum Vorhersagen der Vertikalnährstoffkonzentration des Wasserkörpers in dem Zielgewässer basierend auf der Oberflächennährstoffkonzentration durch eine Beziehungsfunktion zwischen der Oberflächenkonzentration und der Vertikalkonzentration und zum Bestimmen des Ernährungszustands des Wasserkörpers in dem Zielgewässer basierend auf der Oberflächennährstoffkonzentration und der Vertikalnährstoffkonzentration, wobei die Beziehungsfunktion zwischen der Oberflächenkonzentration und der Vertikalkonzentration gemäß der Multiskaleninformation des Wasserkörpers angepasst und bestimmt wird,

ein Wasserblüten-Frühwarnungsmodul (106) zum Vorhersagen der Wahrscheinlichkeit des Auftretens von Wasserblüten gemäß dem Ernährungszustand des Wasserkörpers und zum Erzeugen der entsprechenden Warnrückkopplungsbenachrichtigungsinformation gemäß der Wahrscheinlichkeit des Auftretens von Wasserblüten, und

ein Digitalvisualisierungsmodul (108) zum digitalen Anzeigen der Visualisierung des Ernährungszustands des Wasserkörpers, der Wahrscheinlichkeit des Auftretens von Wasserblüten und der Warnrückkopplungsbenachrichtigungsinformation, wobei das Multiskaleninformation-Erfassungsmodul (102) eine Wasserkörperüberwachungseinheit (200), eine Telemetriedateneinheit (202) und eine Referenzinformationssammeleinheit (204) umfasst,

dass die Wasserkörpersüberwachungseinheit (200) zum Erhalten von Hydrodynamikdaten, Wassertemperaturdaten, Wasserqualitätsdaten und Gewässerwinddaten des Wasserkörpers in dem Zielgewässer ausgebildet ist, dass die physikalische und chemische Information des Wasserkörpers Hydrodynamikdaten, Wassertemperaturdaten, Wasserqualitätsdaten und Gewässerwinddaten des Wasserkörpers umfasst,

dass die Telemetriedateneinheit (202) zum Erhalten von Bilddaten in geringer Höhe und Fernerkundungsdaten in großer Höhe des Zielgewässers ausgebildet ist, dass die Oberflächeninformation des Wasserkörpers die Bilddaten in geringer Höhe und die Fernerkundungsdaten in großer Höhe umfasst,

dass die Referenzinformationssammeleinheit (204) zum Sammeln der entsprechenden Hydrologie- und Hydrodynamikdaten, Gewässerumweltdaten, Topographie- und Geomorphologiedaten sowie Meteorologiedaten des Zielgewässers ausgebildet ist, dass die Referenzhydrologieinformation die Hydrologie- und Hydrodynamikdaten, die Gewässerumweltdaten, die Topographie- und Geomorphologiedaten sowie die Meteorologiedaten umfasst,

dass die Wasserkörpersüberwachungseinheit (200) eine Unterwasser-Multifunktionsdetektionsvorrichtung (300) und eine Überwachungsstation (302) für physikalische und chemische Parameter umfasst,

dass die Unterwasser-Multifunktionsdetektionsvorrichtung (300) zum Erhalten von den Hydrodynamikdaten, den Wassertemperaturdaten und den Wasserqualitätsdaten ausgebildet ist, dass die Überwachungsstation (302) für physikalische und chemische Parameter zum ergänzenden Überwachen der Wasserqualitätsdaten ausgebildet ist,

dass die Unterwasser-Multifunktionsdetektionsvorrichtung (300) eine hydrodynamische Induktionssonde (302), einen Temperatursensor (304) und einen Wasserfarbbildgeber (306) umfasst,

wobei die hydrodynamische Induktionssonde (302) am Unterteil der Unterwasser-Multifunktionsdetektionsvorrichtung (300) angeordnet ist, um hydrodynamische Faktoren mit unterschiedlichen Detektionstiefen zu detektieren und aufzuzeichnen,

dass der Temperatursensor (304) zum Sammeln der Wassertemperaturdaten mit unterschiedlichen Detektionstiefen ausgebildet ist,

dass der Wasserfarbbildgeber (306) zum Fotografieren an verschiedenen Detektionstiefen im Wasserkörper und zum Detektieren und Bestimmen von Sauerstoffauflösungsdaten, Chlorophylldaten, Algendichtedaten, Stickstoffdaten, Phosphordaten und Transparenzdaten im Wasserkörper ausgebildet ist,

wobei der Wasserfarbbildgeber (306) zwei symmetrisch verteilte Wasserfarbbildgebungsplatten umfasst, und außerdem eingerichtet ist, den Abstand zwischen den zwei Wasserfarbbildgebungsplatten gemäß der Detektionstiefe einzustellen,

$$w = \xi \sigma_i \frac{h}{e^\lambda + \eta}$$

dabei $w$ den Abstand zwischen den zwei Wasserfarbbildgebungsplatten darstellt, $\xi$ den Turbulenzkorrekturkoeffizienten darstellt, $\sigma_i$ die Turbulenzintensität des Wasserflusses am $i$ Detektionspunkt darstellt, $h$ die Detektionstiefe entsprechend dem $i$ Detektionspunkt darstellt, $\lambda$ den spektralen Auflösungsfaktor darstellt und $\eta$ den Korrekturparameter darstellt, dass das Ernährungszustand-Erkennungsmodul (104) eine Oberflächenzustandsidentifikationseinheit (400), eine Funktionsanpassungseinheit (402),

eine Vertikalzustandsvorhersageeinheit (404) und eine Zustandsbestimmungseinheit (406) umfasst,

dass die Oberflächenzustandsidentifikationseinheit (400) zum Invertieren und Identifizieren des Ernährungszustands des Oberflächenwasserkörpers in dem Zielgewässer gemäß der Oberflächeninformation des Wasserkörpers ausgebildet ist, um die Oberflächennährstoffkonzentration zu bestimmen,

dass die Oberflächenzustandsidentifikationseinheit (400) ein Datenverbesserungsmodul (4000) umfasst,

dass das (4000) Datenverbesserungsmodul zum Vorhersagen und Berechnen der mit dem fehlenden Periode der Oberflächeninformation des Wasserkörpers entsprechenden Oberflächennährstoffkonzentration bei der Bestimmung der Oberflächennährstoffkonzentration ausgebildet ist,

$$c_{t+\Delta t} = \psi\left(\frac{u}{\sqrt{SD_s}}\Delta t\right)c_t$$

dabei $c_{t+\Delta t}$ die Oberflächennährstoffkonzentration zum Zeitpunkt $t + \Delta t$| darstellt, $c_t$ die Oberflächennährstoffkonzentration zum Zeitpunkt $t$ darstellt, $\Delta t$ die fehlende Zeit darstellt, $\psi$ den Korrekturkoeffizienten der Nährstoffkonzentration darstellt und $S$ die Oberflächenfläche des Zielgewässers darstellt,

dass die Funktionsanpassungseinheit (402) zum Konstruieren des Trainingsdatensatzes unter Verwendung der Multiskaleninformation des Wasserkörpers ausgebildet ist, dass das Training durch einen maschinellen Lernalgorithmus durchgeführt wird, um die Beziehungsfunktion zwischen der Oberflächenkonzentration und der Vertikalkonzentration anzupassen und zu bestimmen,

dass die Beziehungsfunktion zwischen der Oberflächenkonzentration und der Vertikalkonzentration ausgebildet ist, um eine Beziehung zwischen der Nährstoffkonzentration in dem Oberflächenwasserkörper des Zielgewässers und der Nährstoffkonzentration in dem Vertikalwasserkörper des Zielgewässers zu beschreiben,

die Beziehungsfunktion zwischen der Oberflächenkonzentration und der Vertikalkonzentration:

$$F = \left(\mathrm{T}, c_i^s, c_i^n, u, h, D_s\right)$$

dabei $F$ die Beziehungsfunktion zwischen der Oberflächenkonzentration und der Vertikalkonzentration darstellt, T die Wasserkörperstemperatur darstellt, $c_i^s$ die Konzentration des $j$ Nährstoffs in dem Oberflächenwasserkörper darstellt, $c_i^n$ die Konzentration des $j$ Nährstoffs in dem Vertikalwasserkörper darstellt, $u$: die Strömungsgeschwindigkeit des Wasserflusses darstellt, $h$ die Wassertiefe darstellt und $D_s$ den Komponentenausdehnungskoeffizienten darstellt,

dass die Vertikalzustandsvorhersageeinheit (404) zum Durchführen einer Vorhersageberechnung unter Verwendung der Oberflächennährstoffkonzentration als Eingabe gemäß der Beziehungsfunktion zwischen der Oberflächenkonzentration und der Vertikalkonzentration ausgebildet ist, um die Vertikalnährstoffkonzentration zu bestimmen,

dass die Zustandsbestimmungseinheit (406) zum Analysieren und Bestimmen des Ernährungszustands des Wasserkörpers in dem Zielgewässer basierend auf der Oberflächennährstoffkonzentration und der Vertikalnährstoffkonzentration ausgebildet ist,

dass die Zustandsbestimmungseinheit (406) ein Ernährungszustandsindexmodul (4060) und ein Zustandsanalysemodul (4062) umfasst,

dass das Ernährungszustandsindexmodul (4060) zum Berechnen eines entsprechenden umfassenden Ernährungszustandsindex basierend auf Gesamtkonzentrationsdaten verschiedener Nährstoffe in dem Wasserkörper des Zielgewässers ausgebildet ist,

$$TLI = k_1 TLI_{chl-a} + k_2 TLI_{tp} + k_3 TLI_{tn} + k_4 TLI_{sd} + k_5 TLI_{codm-n}$$

dabei $TLI$ den umfassenden Ernährungszustandsindex darstellt, $TLI_{chl-a}$, $TLI_{tp}$, $TLI_{tn}$, $TLI_{sd}$ und $TLI_{codm-n}$ den Zustandsindex entsprechend Chlorophyll a, Phosphor, Stickstoff, Transparenz und Sauerstoffgehalt darstellen und $k_1, k_2, k_3, k_4, k_5$ den Exponentialkoeffizienten darstellt,

dass das Zustandsanalysemodul (4062) zum Bestimmen des Nährstoffgrades des Zielgewässers gemäß dem Carlson-Ernährungszustandsindex ausgebildet ist,

dass das Wasserblüten-Frühwarnungsmodul (106) Algendichtevorhersageeinheit (500) und eine Wasserblütenwahrscheinlichkeitsvorhersageeinheit (502) umfasst,

dass die Algendichtevorhersageeinheit (500) zum Konstruieren eines Algendichtevorhersagemodells durch einen maschinellen Lernalgorithmus ausgebildet ist, dass die Eingabevariable des Algendichtevorhersagemodells den Ernährungszustand des Wasserkörpers umfasst, dass die Ausgabevariable einen Algendichtevorhersagewert umfasst,

dass die Wasserblütenwahrscheinlichkeitsvorhersageeinheit (502) zum Berechnen und Bestimmen der Wahrscheinlichkeit des Auftretens von Wasserblüten basierend auf dem Algendichtevorhersagewert ausgebildet ist.

**2.** System nach Anspruch 1, **dadurch gekennzeichnet, dass** die Funktionsanpassungseinheit (402) ein Kandidatenfunktionsmodul (4020) und ein Funktionsfiltermodul (4022) umfasst,

dass das Kandidatenfunktionsmodul (4020) zum Durchführen eines Lerntrainings gemäß dem Trainingsdatensatz ausgebildet ist, um mehrere Kandidatenbeziehungsfunktionen zu bestimmen,
dass das Funktionsfiltermodul (4022) zum Berechnen und Bestimmen eines Bestimmungskoeffizienten mehrerer Kandidatenbeziehungsfunktionen ausgebildet ist, um eine optimale Funktion als die Beziehungsfunktion zwischen der Oberflächenkonzentration und der Vertikalkonzentration gemäß dem Bestimmungskoeffizienten auszuwählen.

**3.** System nach Anspruch 1, **dadurch gekennzeichnet, dass** das System auch ein Echtzeit-Parameteroptimierungsmodul umfasst,
dass das Echtzeit-Parameteroptimierungsmodul zum dynamischen Optimieren und Aktualisieren eines Identifikationseingabefaktors der Oberflächenzustandsidentifikationseinheit, eines Beziehungsparameters der Beziehungsfunktion zwischen der Oberflächenkonzentration und der Vertikalkonzentration und eines Modellkoeffizienten des Algendichtevorhersagemodells ausgebildet ist.

**4.** Verfahren zur intelligenten Frühwarnung vor Wasserblüten basierend auf der Vorhersage des Ernährungszustands von Gewässern, **dadurch gekennzeichnet, dass** das Verfahren mittels eines Systems nach einem der Ansprüche 1 bis 3 durchgeführt wird und die folgenden Schritte umfasst:

Sammeln der Multiskaleninformation des Wasserkörpers in dem Zielgewässer, wobei die Multiskaleninformation des Wasserkörpers physikalische und chemische Information des Wasserkörpers, Oberflächeninformation des Wasserkörpers und Referenzhydrologieinformation umfasst,
Invertieren und Bestimmen der Oberflächennährstoffkonzentration basierend auf der Oberflächeninformation des Wasserkörpers,
Vorhersagen der Vertikalnährstoffkonzentration des Wasserkörpers in dem Zielgewässer basierend auf der Oberflächennährstoffkonzentration durch eine Beziehungsfunktion zwischen der Oberflächenkonzentration und der Vertikalkonzentration,
Bestimmen des Ernährungszustands des Wasserkörpers in dem Zielgewässer basierend auf der Oberflächennährstoffkonzentration und der Vertikalnährstoffkonzentration, wobei die Beziehungsfunktion zwischen der Oberflächenkonzentration und der Vertikalkonzentration gemäß der Multiskaleninformation des Wasserkörpers angepasst und bestimmt wird,
Vorhersagen der Wahrscheinlichkeit des Auftretens von Wasserblüten gemäß dem Ernährungszustand des Wasserkörpers und Erzeugen der entsprechenden Warnrückkopplungsbenachrichtigungsinformation gemäß der Wahrscheinlichkeit des Auftretens von Wasserblüten, und
digitales Anzeigen der Visualisierung des Ernährungszustands des Wasserkörpers, der Wahrscheinlichkeit des Auftretens von Wasserblüten und der Warnrückkopplungsbenachrichtigungsinformation.

**Claims**

**1.** System (100) for intelligent early warning of algal blooms based on prediction of the nutritional status of waters, **characterized in that** the system comprises the following modules:

a multi-scale information acquisition module (102) for collecting the multi-scale water body information in the target waters, the multi-scale water body information comprising physical and chemical water body information, water body surface information and reference hydrological information,
a nutritional status detection module (104) for inverting and determining the surface nutrient concentration on the basis of the surface information of the water body, for predicting the vertical nutrient concentration of the water body in the target waters on the basis of the surface nutrient concentration by a relationship function between the surface concentration and the vertical concentration, and to determine the nutrient status of the water body in the target waters on the basis of the surface nutrient concentration and the vertical nutrient concentration, the relationship function between surface concentration and vertical concentration being adapted and determined as a function of the multi-scale information of the water body,

an algal bloom early warning module (106) for predicting the probability of occurrence of an algal bloom as a function of the nutritional status of the water body and for generating corresponding early warning notification information as a function of the probability of occurrence of an algal bloom, and

a digital visualization module (108) for digitally displaying the visualization of the nutritional status of the water body, the probability of occurrence of algal blooms and feedback notification information, the multi-scale information acquisition module (102) comprising a water body monitoring unit (200), a telemetry data unit (202) and a reference information collection unit (204),

**in that** the water body monitoring unit (200) is configured to obtain hydrodynamic data, water temperature data, water quality data and water wind data from the water body in the target waters,

**in that** the physical and chemical information for the water body includes hydrodynamic data, water temperature data, water quality data and wind data for the water body,

**in that** the telemetry data unit (202) is configured to obtain low-altitude image data and high-altitude remote sensing data of the target waters,

**in that** the water body surface information comprises low-altitude image data and high-altitude remote sensing data,

**in that** the reference information collection unit (204) is configured to collect the corresponding hydrological and hydrodynamic data, aquatic environment data, topographic and geomorphological data and meteorological data of the target waters, **in that** the reference hydrological information comprises hydrological and hydrodynamic data, aquatic environment data, topographic and geomorphological data and meteorological data,

**in that** the water body monitoring unit (200) comprises an underwater multifunctional detection device (300) and a monitoring station (302) for physical and chemical parameters,

**in that** the underwater multifunctional detection device (300) is configured to obtain hydrodynamic data, water temperature data and water quality data, **in that** the physical and chemical parameter monitoring station (302) is configured to monitor the water quality data in a complementary manner,

**in that** the underwater multifunctional detection device (300) comprises a hydrodynamic induction probe (302), a temperature sensor (304) and a water color imager (306), the hydrodynamic induction probe (302) being arranged on the bottom of the underwater multifunctional detection device (300) for detecting and recording hydrodynamic factors at different detection depths,

**in that** the temperature sensor (304) is configured to collect water temperature data at different detection depths,

**in that** the water color imager (306) is configured to photograph at different detection depths in the water body and to detect and determine oxygen dissolution data, chlorophyll data, algae density data, nitrogen data, phosphorus data and transparency data in the water body,

wherein the water color imager (306) comprises two symmetrically distributed water color imaging plates, and also is configured to adjust the distance between the two water color imaging plates as a function of the detection depth,

$$w = \xi\sigma_i \frac{h}{e^\lambda + \eta}$$

where $\omega$ represents the distance between the two water color imaging plates, ç represents the turbulence correction coefficient, $\sigma_i$ represents the turbulence intensity of the water flow at detection point $i$, $h$ represents the detection depth corresponding to detection point i, $\lambda$ represents the spectral resolution factor and $\eta$ represents the correction parameter,

**in that** the nutritional status detection module (104) comprises a surface status identification unit (400), a function adaptation unit (402), a vertical status prediction unit (404) and a status determination unit (406),

**in that** the surface status identification unit (400) is configured to invert and identify the nutritional status of the surface water body in the target waters according to the surface information of the water body in order to determine the surface nutrient concentration,

**in that** the surface status identification unit (400) comprises a data enhancement module (4000),

**in that** the data enhancement module (4000) is configured to predict and calculate the surface nutrient concentration corresponding to the missing period of the surface information of the water body when determining the surface nutrient concentration,

$$c_{t+\Delta t} = \psi\left(\frac{u}{\sqrt{SD_s}}\Delta t\right)c_t$$

where $C_{t+\Delta t}$ represents the surface nutrient concentration at time $t+\Delta t$, $ct$ represents the surface nutrient concentration at time $t$, $\Delta t$ represents the missing time, $\psi$ represents the nutrient concentration correction coefficient and $S$ represents the surface area of the target waters,

**in that** the function adaptation unit (402) is configured to construct the training data set using the multiscale information of the water body, **in that** the training is performed by a machine learning algorithm in order to fit and determine the relation function between surface concentration and vertical concentration,

**in that** the surface concentration/vertical concentration relationship function is configured to describe a relationship between the nutrient concentration in the surface water body of the target waters and the nutrient concentration in the vertical water body of the target waters,

the relationship function between surface concentration and vertical concentration

$$F = \left(\mathrm{T}, c_j^s, c_j^n, u, h, D_s\right)$$

where F represents the relationship function between surface concentration and vertical concentration, $T$ represents the water body temperature, $C_j^s$ represents the concentration of nutrient $j$ in the surface water body, $C_j^n$ represents the concentration of nutrient $j$ in the vertical water body, $u$ represents the water flow rate, $h$ represents the water depth and $D_s$ represents the coefficient of expansion of the components,

**in that** the vertical status prediction unit (404) is configured to perform a prediction calculation using the surface nutrient concentration as an input in accordance with the relationship function between surface concentration and vertical concentration in order to determine the vertical nutrient concentration,

**in that** the status determination unit (406) is configured to analyze and determine the nutritional status of the water body in the target waters on the basis of the surface nutrient concentration and the vertical nutrient concentration,

**in that** the status determination unit (406) comprises a nutritional status index module (4060) and a status analysis module (4062),

**in that** the nutritional status index module (4060) is configured to calculate a corresponding overall nutritional status index on the basis of data on the total concentration of various nutrients in the water body of the target waters,

$$TLI = k_1 TLI_{chl-a} + k_2 TLI_{tp} + k_3 TLI_{tn} + k_4 TLI_{sd} + k_5 TLI_{codm-n}$$

where $TLI$ represents the overall nutritional status index, $TLI_{chl-a}$, $TLI_{tp}$, $TLI_{tn}$, $TLI_{sd}$ and $TLI_{codm-n}$ represent the status index corresponding to chlorophyll a, phosphorus, nitrogen, transparency and oxygen content, and $k_1$, $k_2$, $k_3$, $k_4$, $k_5$ represent the exponential coefficients,

**in that** the condition analysis module (4062) is configured to determine the nutrient level of the target waters as a function of the Carlson nutritional condition index, **in that** the algal bloom early warning module (106) comprises an algal density prediction unit (500) and an algal bloom probability prediction unit (502),

**in that** the algae density prediction unit (500) is configured to build an algae density prediction model by a machine learning algorithm, **in that** the input variable of the algae density prediction model comprises the nutritional status of the water body, **in that** the output variable comprises an algae density prediction value,

**in that** the algae bloom probability prediction unit (502) is configured to calculate and determine the probability of algae bloom occurrence on the basis of the algae density prediction value.

2. System according to claim 1, **characterized in that** the function adaptation unit (402) comprises a candidate function module (4020) and a function filter module (4022),

**in that** the candidate function module (4020) is configured to perform training based on the training data set to determine a plurality of candidate relationship functions,

**in that** the function filter module (4022) is configured to calculate and determine a coefficient of determination of several candidate relationship functions in order to select an optimum function as the relationship function between surface concentration and vertical concentration according to the coefficient of determination.

3. System according to claim 1, **characterized in that** the system also comprises a real-time parameter optimization module,

   **in that** the real-time parameter optimization module is configured to dynamically optimize and update an identification input factor of the surface status identification unit, a relationship parameter of the relationship function between surface concentration and vertical concentration, and a model coefficient of the algae density prediction model.

4. Method of intelligent early warning of algal blooms based on prediction of the nutritional status of waters, **characterized in that** the method is carried out by means of a system according to any one of claims 1 to 3 and comprises the following steps:

   collecting multi-scale water body information in target waters, wherein multi-scale water body information comprises physical and chemical water body information, water body surface information and reference hydrological information,
   inverting and determining surface nutrient concentration based on water body surface information,
   predicting the vertical nutrient concentration of the water body in the target waters on the basis of the surface nutrient concentration by means of a relationship function between surface concentration and vertical concentration,
   determining the nutritional status of the water body in target waters on the basis of surface nutrient concentration and vertical nutrient concentration, with the relationship function between surface concentration and vertical concentration being adapted and determined on the basis of multi-scale information on the water body,
   predicting the probability of algal bloom occurrence as a function of the nutritional status of the water body, and generating corresponding early warning notification information as a function of the probability of algal bloom occurrence, and
   digitally displaying of the nutritional status of the water body, the probability of the occurrence of algal blooms and notification of alerts.

## Revendications

1. Système (100) d'avertissement précoce intelligent d'efflorescence algale basé sur la prédiction de l'état nutritionnel d'eaux, **caractérisé en ce que** le système comprend les modules suivants :

   un module d'acquisition d'informations multi-échelles (102) pour collecter les informations multi-échelles de la masse d'eau dans les eaux cibles, les informations multi-échelles de la masse d'eau comprenant des informations physiques et chimiques de la masse d'eau, des informations de surface de la masse d'eau et des informations hydrologiques de référence,
   un module de détection d'état nutritionnel (104) pour inverser et déterminer la concentration en nutriments de surface sur la base des informations de surface de la masse d'eau, pour prédire la concentration verticale en nutriments de la masse d'eau dans les eaux cibles sur la base de la concentration en nutriments de surface par une fonction de relation entre la concentration de surface et la concentration verticale, et pour déterminer l'état nutritionnel de la masse d'eau dans les eaux cibles sur la base de la concentration en nutriments de surface et de la concentration verticale en nutriments, la fonction de relation entre la concentration de surface et la concentration verticale étant adaptée et déterminée en fonction des informations multi-échelles de la masse d'eau,
   un module d'alerte précoce d'efflorescence algale (106) pour prédire la probabilité d'apparition d'efflorescence algale en fonction de l'état nutritionnel de la masse d'eau et pour générer les informations de notification de retour d'alerte correspondantes en fonction de la probabilité d'apparition d'efflorescence algale, et
   un module de visualisation numérique (108) pour afficher numériquement la visualisation de l'état nutritionnel de la masse d'eau, la probabilité d'apparition d'efflorescence algale et les informations de notification de retour d'alerte, le module d'acquisition d'informations multi-échelles (102) comprenant une unité de surveillance de masse d'eau (200), une unité de données de télémétrie (202) et une unité de collecte d'informations de référence (204),
   **en ce que** l'unité de surveillance de masse d'eau (200) est configurée pour obtenir des données hydrodynamiques, des données de température d'eau, des données de qualité d'eau et des données de vent d'eau de la masse d'eau dans les eaux cibles,

**en ce que** les informations physiques et chimiques de la masse d'eau comprennent des données hydrodynamiques, des données de température d'eau, des données de qualité d'eau et des données de vent d'eau de la masse d'eau,

**en ce que** l'unité de données de télémétrie (202) est configurée pour obtenir des données d'image à basse altitude et des données de télédétection à haute altitude des eaux cibles,

**en ce que** les informations de surface de la masse d'eau comprennent les données d'image à basse altitude et les données de télédétection à haute altitude,

**en ce que** l'unité de collecte d'informations de référence (204) est configurée pour collecter les données hydrologiques et hydrodynamiques, les données d'environnement aquatique, les données topographiques et géomorphologiques et les données météorologiques correspondantes des eaux cibles, **en ce que** les informations hydrologiques de référence comprennent les données hydrologiques et hydrodynamiques, les données sur l'environnement aquatique, les données topographiques et géomorphologiques et les données météorologiques,

**en ce que** l'unité de surveillance de la masse d'eau (200) comprend un dispositif de détection multifonctionnel sous-marin (300) et une station de surveillance (302) des paramètres physiques et chimiques,

**en ce que** le dispositif de détection multifonctionnel sous-marin (300) est configuré pour obtenir les données hydrodynamiques, les données de température de l'eau et les données de qualité de l'eau, **en ce que** la station de surveillance (302) de paramètres physiques et chimiques est configurée pour surveiller de manière complémentaire les données de qualité de l'eau,

**en ce que** le dispositif de détection multifonctionnel sous-marin (300) comprend une sonde d'induction hydrodynamique (302), un capteur de température (304) et un imageur de couleur de l'eau (306), la sonde d'induction hydrodynamique (302) étant agencée sur la partie inférieure du dispositif de détection multifonctionnel sous-marin (300) pour détecter et enregistrer des facteurs hydrodynamiques à différentes profondeurs de détection,

**en ce que** le capteur de température (304) est configuré pour collecter les données de température de l'eau à différentes profondeurs de détection,

**en ce que** l'imageur de couleur de l'eau (306) est configuré pour photographier à différentes profondeurs de détection dans la masse d'eau et pour détecter et déterminer les données de dissolution de l'oxygène, les données de chlorophylle, les données de densité des algues, les données d'azote, les données de phosphore et les données de transparence dans la masse d'eau,

l'imageur de couleur de l'eau (306) comprenant deux plaques d'imagerie de couleur de l'eau réparties symétriquement, et étant également conçu pour ajuster la distance entre les deux plaques d'imagerie de couleur de l'eau en fonction de la profondeur de détection,

$$w = \xi \sigma_i \frac{h}{e^\lambda + \eta}$$

où $\omega$ représente la distance entre les deux plaques d'imagerie de couleur de l'eau, $\xi$ représente le coefficient de correction de la turbulence, $\sigma_i$ représente l'intensité de la turbulence de l'écoulement d'eau au point de détection $i$, $h$ représente la profondeur de détection correspondant au point de détection $i$, $\lambda$ représente le facteur de résolution spectrale et $\eta$ représente le paramètre de correction,

**en ce que** le module de détection d'état nutritionnel (104) comprend une unité d'identification d'état de surface (400), une unité d'adaptation de fonction (402),une unité de prédiction d'état vertical (404) et une unité de détermination d'état (406),

**en ce que** l'unité d'identification d'état de surface (400) est configurée pour inverser et identifier l'état nutritionnel de la masse d'eau de surface dans les eaux cibles selon les informations de surface de la masse d'eau afin de déterminer la concentration en nutriments de surface,

**en ce que** l'unité d'identification d'état de surface (400) comprend un module d'amélioration de données (4000),

**en ce que** le module d'amélioration de données (4000) est configuré pour prédire et calculer la concentration en nutriments de surface correspondant à la période manquante des informations de surface de la masse d'eau lors de la détermination de la concentration en nutriments de surface,

$$c_{t+\Delta t} = \psi\left(\frac{u}{\sqrt{SD_s}}\Delta t\right)c_t$$

où $C_{t+\Delta t}$ représente la concentration en nutriments de surface à l'instant $t+\Delta t$, $c_t$ représente la concentration en nutriments de surface à l'instant $t$, $\Delta t$ représente le temps manquant, $\psi$ représente le coefficient de correction de la concentration en nutriments et $S$ représente l'aire de surface des eaux cibles,

**en ce que** l'unité d'adaptation de fonction (402) est configurée pour construire l'ensemble de données d'apprentissage en utilisant les informations multi-échelle de la masse d'eau, **en ce que** l'apprentissage est effectué par un algorithme d'apprentissage automatique afin d'ajuster et de déterminer la fonction de relation entre la concentration de surface et la concentration verticale,

**en ce que** la fonction de relation entre la concentration de surface et la concentration verticale est configurée pour décrire une relation entre la concentration de nutriments dans la masse d'eau de surface des eaux cibles et la concentration de nutriments dans la masse d'eau verticale des eaux cibles,

la fonction de relation entre la concentration de surface et la concentration verticale

$$F = \left(T, c_j^s, c_j^n, u, h, D_s\right)$$

où F représente la fonction de relation entre la concentration de surface et la concentration verticale, $T$ représente la température de la masse d'eau, $C_j^s$ représente la concentration du nutriment $j$ dans la masse d'eau de surface, $C_j^n$ représente la concentration du nutriment $j$ dans la masse d'eau verticale, $u$ représente le débit de l'écoulement d'eau, $h$ représente la profondeur de l'eau et $D_s$ représente le coefficient de dilatation des composants,

**en ce que** l'unité de prédiction d'état vertical (404) est configurée pour effectuer un calcul de prédiction en utilisant la concentration en nutriments de surface en tant qu'entrée conformément à la fonction de relation entre la concentration de surface et la concentration verticale afin de déterminer la concentration verticale en nutriments,

**en ce que** l'unité de détermination d'état (406) est configurée pour analyser et déterminer l'état nutritionnel de la masse d'eau dans les eaux cibles sur la base de la concentration en nutriments de surface et de la concentration verticale en nutriments,

**en ce que** l'unité de détermination d'état (406) comprend un module d'indice d'état nutritionnel (4060) et un module d'analyse d'état (4062),

**en ce que** le module d'indice d'état nutritionnel (4060) est configuré pour calculer un indice d'état nutritionnel global correspondant sur la base de données de concentration totale de différents nutriments dans la masse d'eau des eaux cibles,

$$TLI = k_1 TLI_{chl-a} + k_2 TLI_{tp} + k_3 TLI_{tn} + k_4 TLI_{sd} + k_5 TLI_{codm-n}$$

où $TLI$ représente l'indice d'état nutritionnel global, $TLI_{chl-a}$, $TLI_{tp}$, $TLI_{tn}$, $TLI_{sd}$ et $TLI_{codm-n}$ représentent l'indice d'état correspondant à la chlorophylle a, au phosphore, à l'azote, à la transparence et à la teneur en oxygène, et $k_1$, $k_2$, $k_3$, $k_4$, $k_5$ représentent les coefficients exponentiels,

**en ce que** le module d'analyse d'état (4062) est configuré pour déterminer le niveau de nutriments des eaux cibles en fonction de l'indice d'état nutritionnel de Carlson, **en ce que** le module d'alerte précoce d'efflorescence algale (106) comprenant une unité de prédiction de la densité des algues (500) et une unité de prédiction de la probabilité d'efflorescence algale (502),

**en ce que** l'unité de prédiction de la densité des algues (500) est configurée pour construire un modèle de prédiction de la densité des algues par un algorithme d'apprentissage automatique, **en ce que** la variable d'entrée du modèle de prédiction de la densité des algues comprend l'état nutritionnel de la masse d'eau, **en ce que** la variable de sortie comprend une valeur de prédiction de la densité des algues,

**en ce que** l'unité de prédiction de la probabilité d'efflorescence algale (502) est configurée pour calculer et déterminer la probabilité d'apparition d'efflorescence algale sur la base de la valeur de prédiction de la densité des algues.

**2.** Système selon la revendication 1, **caractérisé en ce que** l'unité d'adaptation de fonction (402) comprend un module de fonction candidate (4020) et un module de filtre de fonction (4022),

> **en ce que** le module de fonction candidate (4020) est configuré pour effectuer un apprentissage en fonction de l'ensemble de données d'apprentissage afin de déterminer plusieurs fonctions de relation candidates,
> **en ce que** le module de filtre de fonction (4022) est configuré pour calculer et déterminer un coefficient de détermination de plusieurs fonctions de relation candidates afin de sélectionner une fonction optimale en tant que fonction de relation entre la concentration de surface et la concentration verticale selon le coefficient de détermination.

**3.** Système selon la revendication 1, **caractérisé en ce que** le système comprend également un module d'optimisation de paramètres en temps réel,
**en ce que** le module d'optimisation de paramètres en temps réel est configuré pour optimiser et mettre à jour de manière dynamique un facteur d'entrée d'identification de l'unité d'identification d'état de surface, un paramètre de relation de la fonction de relation entre la concentration de surface et la concentration verticale, et un coefficient de modèle du modèle de prédiction de la densité des algues.

**4.** Procédé d'alerte précoce intelligente d'efflorescence algale basé sur la prédiction de l'état nutritionnel d'eaux, **caractérisé en ce que** le procédé est effectué au moyen d'un système selon l'une quelconque des revendications 1 à 3 et comprend les étapes suivantes :

> la collecte des informations multi-échelles de la masse d'eau dans les eaux cibles, les informations multi-échelles de la masse d'eau comprenant des informations physiques et chimiques de la masse d'eau, des informations de surface de la masse d'eau et des informations hydrologiques de référence,
> l'inversion et la détermination de la concentration en nutriments de surface sur la base des informations de surface de la masse d'eau,
> la prédiction de la concentration verticale en nutriments de la masse d'eau dans les eaux cibles sur la base de la concentration en nutriments de surface par une fonction de relation entre la concentration de surface et la concentration verticale,
> la détermination de l'état nutritionnel de la masse d'eau dans les eaux cibles sur la base de la concentration en nutriments de surface et de la concentration verticale en nutriments, la fonction de relation entre la concentration de surface et la concentration verticale étant adaptée et déterminée en fonction des informations multi-échelles de la masse d'eau,
> la prédiction de la probabilité d'apparition d'efflorescence algale en fonction de l'état nutritionnel de la masse d'eau et la génération des informations de notification de retour d'alerte correspondantes en fonction de la probabilité d'apparition d'efflorescence algale, et
> l'affichage numérique de la visualisation de l'état nutritionnel de la masse d'eau, de la probabilité d'apparition d'efflorescence algale et des informations de notification de retour d'alerte.

System zur intelligenten Frühwarnung ~~von~~ vor Wasserblüten basierend auf der Vorhersage des Ernährungszustands von Gewässern

| Modul zum Sammeln der Multiskaleninformation 102 | Modul zum Identifizieren des Ernährungs-zustands 104 | Modul zur Frühwarnung ~~von~~ vor Wasserblüten 106 |

Modul zur digitalen Visualisierung 108

**100**

Figur 1

Wasserkörpersüberwachungseinheit

Unterwasser-Multifunktions-detektionsvorrichtung 300

Telemetriedateneinheit 202

Überwachungsstation für physikalische und chemische Parameter 302

Referenzinformations-sammeleinheit 204

200

Modul zum Sammeln der Multiskaleninformation 102

Figur 2

306

304

300

302

Figur 3

| Modul zum Identifizieren des Ernährungszustands | | | |
|---|---|---|---|
| Oberflächenzustands-identifikationseinheit | Funktions-anpassungseinheit | Vertikal-zustands-vorhersage-einheit | Zustands-bestimmungseinheit |
| Daten-perfektions-modul 4000 | Kandidaten-funktionsmodul 4020 / Funktionsfilter-modul 4022 | | Ernährungs-zustandsindex-modul 4060 / Zustandsanalyse-modul 4062 |
| 400 | 402 | 404 | 406 |

104

Figur 4

Modul zur Frühwarnung ~~von~~ vor Wasserblüten

Algendichtevorhersageeinheit
500

Wasserblütenwahrscheinlichkeitsvorhersageeinheit 502

106

Figur 5

Figur 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 111242380